(19) Europäisches Patentamt
European Patent Office
Office européen des brevets

(11) EP 1 574 502 A1

(12) EUROPEAN PATENT APPLICATION

(43) Date of publication:
14.09.2005 Bulletin 2005/37

(51) Int Cl.[7]: C07D 239/46, C07D 239/60, C07D 239/50

(21) Application number: 04005423.1

(22) Date of filing: 08.03.2004

(84) Designated Contracting States:
AT BE BG CH CY CZ DE DK EE ES FI FR GB GR HU IE IT LI LU MC NL PL PT RO SE SI SK TR
Designated Extension States:
AL LT LV MK

(71) Applicant: Novartis AG
4002 Basel (CH)

(72) Inventor: The designation of the inventor has not yet been filed

(74) Representative: Gros, Florent et al
Novartis AG,
Corporate Intellectual Property
4002 Basel (CH)

Remarks:
Claims 11 - 26 are deemed to be abandoned due to non-payment of the claims fees (Rule 31 (2) EPC).

(54) **Use of pyrimidine compounds in the preparation of parasiticides**

(57) The invention relates to the use of compounds of the general formula

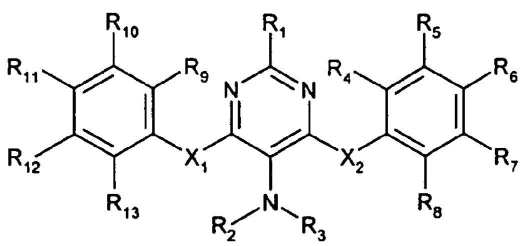

I,

wherein $R_1$, $R_2$, $R_3$, $R_4$, $R_5$, $R_6$, $R_7$, $R_8$, $R_9$, $R_{10}$, $R_{11}$, $R_{12}$, $R_{13}$, $X_1$ and $X_2$ have the significances given in claim 1, and optionally the enantiomers and geometrical isomers thereof, for controlling parasites on warm-blooded animals.

EP 1 574 502 A1

## Description

The present invention relates to the use of 4,6-disubstituted 5-aminopyrimidine compounds of formula

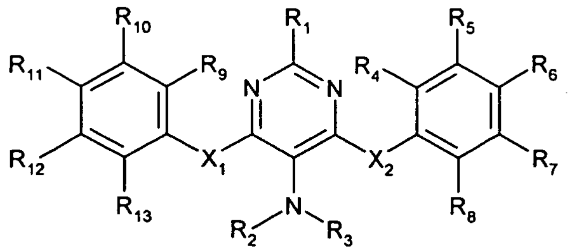

I,

wherein

$R_1$ is hydrogen, halogen, cyano, OH, SH, $NO_2$, COOH, $COOR_2$, $CONH_2$, $CONR_2R_3$, $SO_3H$, $SO_2NR_2R_3$, $C_1$-$C_6$-alkyl, halo-$C_1$-$C_6$-alkyl, $C_1$-$C_6$-alkoxy, halo-$C_1$-$C_6$-alkoxy, $C_2$-$C_6$-alkenyl, halo-$C_2$-$C_6$-alkenyl, $C_2$-$C_6$-alkinyl, $C_3$-$C_6$-cycloalkyl, halo-$C_3$-$C_6$-cycloalkyl, $C_3$-$C_6$-cyclo-alkyloxy, $C_3$-$C_6$-cycloalkylthio, $C_2$-$C_6$-alkenyloxy, halo-$C_2$-$C_6$-alkenyloxy, $C_1$-$C_6$-alkylthio, halo-$C_1$-$C_6$-alkylthio, $C_1$-$C_6$-alkylsulfonyloxy, halo-$C_1$-$C_6$-alkylsulfonyloxy, $C_1$-$C_6$-alkylsulfinyl, halo-$C_1$-$C_6$-alkylsulfinyl, $C_1$-$C_6$-alkylsulfonyl, halo-$C_1$-$C_6$-alkylsulfonyl, $C_2$-$C_6$-alkenylthio, halo-$C_2$-$C_6$-alkenylthio, $C_2$-$C_6$-alkenylsulfinyl, halo-$C_2$-$C_6$-alkenylsulfinyl, $C_2$-$C_6$-alkenylsulfonyl, halo-$C_2$-$C_6$-alkenylsulfonyl, $NR_2R_3$, unsubstituted or one- to five-fold substituted aryl or unsubstituted or substituted hetaryl, the substituents selected from the group consisting of halogen, cyano, OH, SH, $NO_2$, COOH, $COOR_2$, $CONH_2$, $CONR_2R_3$, $SO_3H$, $SO_2NR_2R_3$, $C_1$-$C_6$-alkyl, halo-$C_1$-$C_6$-alkyl, $C_1$-$C_6$-alkoxy, halo-$C_1$-$C_6$-alkoxy, $C_2$-$C_6$-alkenyl, halo-$C_2$-$C_6$-alkenyl, $C_2$-$C_6$-alkinyl, $C_3$-$C_6$-cycloalkyl, halo-$C_3$-$C_6$-cycloalkyl, $C_3$-$C_6$-cycloalkyloxy, $C_3$-$C_6$-cycloalkylthio, $C_2$-$C_6$-alkenyloxy, halo-$C_2$-$C_6$-alkenyloxy, $C_1$-$C_6$-alkylthio, halo-$C_1$-$C_6$-alkylthio, $C_1$-$C_6$-alkylsulfonyloxy, halo-$C_1$-$C_6$-alkylsulfonyloxy, $C_1$-$C_6$-alkylsulfinyl, halo-$C_1$-$C_6$-alkylsulfinyl, $C_1$-$C_6$-alkylsulfonyl, halo-$C_1$-$C_6$-alkylsulfonyl, $C_2$-$C_6$-alkenylthio, halo-$C_2$-$C_6$-alkenylthio, $C_2$-$C_6$-alkenylsulfinyl, halo-$C_2$-$C_6$-alkenylsulfinyl, $C_2$-$C_6$-alkenylsulfonyl, halo-$C_2$-$C_6$-alkenylsulfonyl and $NR_2R_3$;

$R_2$ and $R_3$, independently of one another, signify hydrogen, $C_1$-$C_6$-alkyl, halo-$C_1$-$C_6$-alkyl, formyl, $C_1$-$C_6$-alkylcarbonyl, halo-$C_1$-$C_6$-alkylcarbonyl, $C_1$-$C_6$-alkoxycarbonyl, halo-$C_1$-$C_6$-alkoxycarbonyl, $C_1$-$C_6$-alkylaminocarbonyl, di-$C_1$-$C_6$-alkylaminocarbonyl or unsubstituted or one- to five-fold substituted benzyl, the substituents selected from the group consisting of halogen, cyano, OH, SH, $NO_2$, COOH, $COOR_2$, $CONH_2$, $CONR_2R_3$, $SO_3H$, $SO_2NR_2R_3$, $C_1$-$C_6$-alkyl, halo-$C_1$-$C_6$-alkyl, $C_1$-$C_6$-alkoxy, halo-$C_1$-$C_6$-alkoxy, $C_2$-$C_6$-alkenyl, halo-$C_2$-$C_6$-alkenyl, $C_2$-$C_6$-alkinyl, $C_3$-$C_6$-cycloalkyl, halo-$C_3$-$C_6$-cycloalkyl, $C_3$-$C_6$-cycloalkyloxy, $C_3$-$C_6$-cycloalkylthio, $C_2$-$C_6$-alkenyloxy, halo-$C_2$-$C_6$-alkenyloxy, $C_1$-$C_6$-alkylthio, halo-$C_1$-$C_6$-alkylthio, $C_1$-$C_6$-alkylsulfonyloxy, halo-$C_1$-$C_6$-alkylsulfonyloxy, $C_1$-$C_6$-alkylsulfinyl, halo-$C_1$-$C_6$-alkylsulfinyl, $C_1$-$C_6$-alkylsulfonyl, halo-$C_1$-$C_6$-alkylsulfonyl, $C_2$-$C_6$-alkenylthio, halo-$C_2$-$C_6$-alkenylthio, $C_2$-$C_6$-alkenylsulfinyl, halo-$C_2$-$C_6$-alkenylsulfinyl, $C_2$-$C_6$-alkenylsulfonyl and halo-$C_2$-$C_6$-alkenylsulfonyl;

$R_4$, $R_5$, $R_6$, $R_7$, $R_8$, $R_9$, $R_{10}$, $R_{11}$, $R_{12}$ and $R_{13}$, independently of one another, are hydrogen, halogen, cyano, nitro, OH, SH, $NO_2$, COOH, $COOR_2$, $CONH_2$, $CONR_2R_3$, $SO_3H$, $SO_2NR_2R_3$, $C_1$-$C_6$-alkyl, halo-$C_1$-$C_6$-alkyl, $C_1$-$C_6$-alkoxy, halo-$C_1$-$C_6$-alkoxy, $C_2$-$C_6$-alkenyl, halo-$C_2$-$C_6$-alkenyl, $C_2$-$C_6$-alkinyl, $C_3$-$C_6$-cycloalkyl, $C_2$-$C_6$-alkenyloxy, halo-$C_2$-$C_6$-alkenyloxy, $C_1$-$C_6$-alkylthio, halo-$C_1$-$C_6$-alkylthio, $C_1$-$C_6$-alkylsulfonyloxy, halo-$C_1$-$C_6$-alkylsulfonyloxy, $C_1$-$C_6$-alkylsulfinyl, halo-$C_1$-$C_6$-alkylsulfinyl, $C_1$-$C_6$-alkylsulfonyl, halo-$C_1$-$C_6$-alkylsulfonyl, $C_2$-$C_6$-alkenylthio, halo-$C_2$-$C_6$-alkenylthio, $C_2$-$C_6$-alkenylsulfinyl, halo-$C_2$-$C_6$-alkenylsulfinyl, $C_2$-$C_6$-alkenylsulfonyl, halo-$C_2$-$C_6$-alkenylsulfonyl, $C_1$-$C_6$-alkylamino, di-$C_1$-$C_6$-alkylamino, $C_1$-$C_6$-alkylsulfonylamino, halo-$C_1$-$C_6$-alkylsulfonylamino, $C_1$-$C_6$-alkylcarbonyl, halo-$C_1$-$C_6$-alkylcarbonyl, $C_1$-$C_6$-alkoxycarbonyl, $C_1$-$C_6$-alkylaminocarbonyl, di-$C_1$-$C_6$-alkylaminocarbonyl, unsubstituted or one- to five-fold substituted aryl or unsubstituted or substituted hetaryl, the substituents selected from the group consisting of halogen, cyano, OH, SH, $NO_2$, COOH, $COOR_2$, $CONH_2$, $CONR_2R_3$, $SO_3H$, $SO_2NR_2R_3$, $C_1$-$C_6$-alkyl, halo-$C_1$-$C_6$-alkyl, $C_1$-$C_6$-alkoxy, halo-$C_1$-$C_6$-alkoxy, $C_2$-$C_6$-alkenyl, halo-$C_2$-$C_6$-alkenyl, $C_2$-$C_6$-alkinyl, $C_3$-$C_6$-cycloalkyl, halo-$C_3$-$C_6$-cycloalkyl, $C_3$-$C_6$-cycloalkyloxy, $C_3$-$C_6$-cycloalkylthio, $C_2$-$C_6$-alkenyloxy, halo-$C_2$-$C_6$-alkenyloxy, $C_1$-$C_6$-alkylthio, halo-$C_1$-$C_6$-alkylthio, $C_1$-$C_6$-alkylsulfonyloxy, halo-$C_1$-$C_6$-alkylsulfonyloxy, $C_1$-$C_6$-alkylsulfinyl, halo-$C_1$-$C_6$-alkylsulfinyl, $C_1$-$C_6$-alkylsulfonyl, halo-$C_1$-$C_6$-alkylsulfonyl, $C_2$-$C_6$-alkenylthio, halo-$C_2$-$C_6$-alkenylthio, $C_2$-$C_6$-alkenylsulfinyl, halo-$C_2$-$C_6$-alkenylsulfinyl, $C_2$-$C_6$-alkenylsulfonyl, halo-$C_2$-$C_6$-alkenylsulfonyl and $NR_2R_3$;

$X_1$ and $X_2$, independently of one another, are $C(R_{14})(R_{15})$, $N(R_{14})(R_{15})$, O, S, SO or $SO_2$ and

$R_{14}$ and $R_{15}$, independently of one another, signify hydrogen, $C_1$-$C_6$-alkyl, formyl, $C_1$-$C_6$-alkylcarbonyl or halo-$C_1$-$C_6$-alkylcarbonyl;

in the control of ectoparasites, especially ticks, on non-human animals, especially productive livestock and domestic animals, furthermore pesticidal compositions which contain at least one of these compounds.

In literature, e. g. WO9854154, WO0049001, WO0224663 or US6342499, various compounds have been proposed as active ingredients having pesticidal properties for use on domestic animals. The biological properties of these known compounds, however, are not fully satisfactory in the field of pest control, which is why there is a need to produce further compounds with pesticidal properties, especially for the control of ectoparasites; this problem is solved according to the invention with the usage of the present compounds I.

Alkyl - as a group per se and as structural element of other groups and compounds such as halogen-alkyl, alkylamino, alkoxy, alkylthio, alkylsulfinyl and alkylsulfonyl - is, in each case with due consideration of the specific number of carbon atoms in the group or compound in question, either straight-chained, i.e. methyl, ethyl, propyl, butyl, pentyl, hexyl, heptyl or octyl, or branched, e.g. isopropyl, isobutyl, sec.-butyl, tert.-butyl, isopentyl, neopentyl or isohexyl.

Cycloalkyl - as a group *per se* and as structural element of other groups and compounds such as halocycloalkyl, cycloalkoxy and cycloalkylthio, - is, in each case with due consideration of the specific number of carbon atoms in the group or compound in question, cyclopropyl, cyclobutyl, cyclopentyl, cyclohexyl, cycloheptyl or cyclooctyl.

Alkenyl - as a group per se and as structural element of other groups and compounds - is, in each case with due consideration of the specific number of carbon atoms in the group or compound in question and of the conjugated or isolated double bonds - either straight-chained, e.g. allyl, 2-butenyl, 3-pentenyl, 1-hexenyl, 1-heptenyl, 1,3-hexadienyl or 1,3-octadienyl, or branched, e.g. isopropenyl, isobutenyl, isoprenyl, tert.-pentenyl, isohexenyl, isoheptenyl or isooctenyl.

Alkinyl - as a group *per se* and as structural element of other groups and compounds - is, in each case with due consideration of the specific number of carbon atoms in the group or compound in question and of the conjugated or isolated double bonds - either straight-chained, e.g. propargyl, 2-butinyl, 3-pentinyl, 1-hexinyl, 1-heptinyl, 3-hexen-1-inyl or 1,5-heptadien-3-inyl, or branched, e.g. 3-methylbut-1-inyl, 4-ethylpent-1-inyl, 4-methylhex-2-inyl or 2-methylhept-3-inyl.

Aryl is phenyl or naphtyl.

Hetaryl is pyridyl, pyrimidyl, s-triazinyl, 1,2,4-triazinyl, thienyl, furanyl, pyrryl, pyrazolyl, imidazolyl, thiazolyl, triazolyl, oxazolyl, thiadiazolyl, oxadiazolyl, benzothienyl, benzofuranyl, benzothiazolyl, indolyl or indazolyl, preferably pyridyl, pyrimidyl, pyrryl, imidazolyl or furanyl, in particular pyridyl or pyrimidyl.

As a rule, halogen signifies fluorine, chlorine, bromine or iodine. The same applies to halogen in combination with other significances, such as halogenalkyl.

Halogen-substituted carbon-containing groups and compounds may be partially halogenated or perhalogenated, whereby in the case of multiple halogenation, the halogen substituents may be identical or different. Examples of halogen-alkyl - as a group *per se* and as structural element of other groups and compounds such as halogen-alkoxy or halogen-alkylthio, - are methyl which is mono- to trisubstituted by fluorine, chlorine and/or bromine, such as $CHF_2$ or $CF_3$; ethyl which is mono- to pentasubstituted by fluorine, chlorine and/or bromine, such as $CH_2CF_3$, $CF_2CF_3$, $CF_2CCl_3$, $CF_2CHCl_2$, $CF_2CHF_2$, $CF_2CFCl_2$, $CF_2CHBr_2$, $CF_2CHClF$, $CF_2CHBrF$ or $CClFCHClF$; propyl or isopropyl, mono- to heptasubstituted by fluorine, chlorine and/or bromine, such as $CH_2CHBrCH_2Br$, $CF_2CHFCF_3$, $CH_2CF_2CF_3$ or $CH(CF_3)_2$; butyl or one of its isomers, mono- to nonasubstituted by fluorine, chlorine and/or bromine, such as $CF(CF_3)CHFCF_3$ or $CH_2(CF_2)_2CF_3$; pentyl or one of its isomers substituted once to eleven times by fluorine, chlorine and/or bromine, such as $CF(CF_3)(CHF)_2CF_3$ or $CH_2(CF_2)_3CF_3$; and hexyl or one of its isomers substituted once to thirteen times by fluorine, chlorine and/or bromine, such as $(CH_2)_4CHBrCH_2Br$, $CF_2(CHF)_4CF_3$, $CH_2(CF_2)_4CF_3$ or $C(CF_3)_2(CHF)_2CF_3$.

Alkoxy groups preferably have a chain length of 1 to 6 carbon atoms. Alkoxy is for example methoxy, ethoxy, propoxy, isopropoxy, n-butoxy, isobutoxy, sec.-butoxy and tert.-butoxy, as well as the isomers pentyloxy and hexyloxy; preferably methoxy and ethoxy. Halogenalkoxy groups preferably have a chain length of 1 to 6 carbon atoms. Halogenalkoxy is e.g. fluoromethoxy, difluoromethoxy, trifluoromethoxy, 2,2,2-trifluoroethoxy, 1,1,2,2-tetrafluoroethoxy, 2-fluoroethoxy, 2-chloroethoxy, 2,2-difluoroethoxy and 2,2,2-trichloroethoxy; preferably difluoromethoxy, 2-chloroethoxy and trifluoromethoxy.

Preferred embodiments within the scope of the invention are:

(1) A compound of formula I, wherein

$R_1$ is hydrogen, halogen, $C_1$-$C_6$-alkyl, halo-$C_1$-$C_6$-alkyl, $C_1$-$C_6$-alkoxy, halo-$C_1$-$C_6$-alkoxy, $C_3$-$C_6$-cycloalkyl,

halo-$C_3$-$C_6$-Cycloalkyl, $C_3$-$C_6$-cycloalkyloxy, $C_3$-$C_6$-cycloalkylthio, $C_1$-$C_6$-alkylthio or halo-$C_1$-$C_6$-alkylthio or, unsubstituted or one- to five-fold substituted aryl or unsubstituted or substituted hetaryl,;

especially hydrogen, halogen, $C_1$-$C_6$-alkyl, halo-$C_1$-$C_6$-alkyl, $C_1$-$C_6$-alkoxy or halo-$C_1$-$C_6$-alkoxy;

particularly hydrogen, $C_1$-$C_6$-alkyl or $C_1$-$C_6$-alkoxy;

(2) A compound of formula I, wherein

$R_2$ and $R_3$, independently of one another, signify hydrogen, $C_1$-$C_6$-alkyl, formyl, $C_1$-$C_6$-alkylcarbonyl, $C_1$-$C_6$-alkoxycarbonyl, $C_1$-$C_6$-alkylaminocarbonyl, di-$C_1$-$C_6$-alkylaminocarbonyl or unsubstituted or one- to five-fold substituted benzyl,;

especially, independently of one another, hydrogen, $C_1$-$C_4$-alkyl, formyl, $C_1$-$C_4$-alkylcarbonyl or benzyl;

particularly, independently of one another, hydrogen, $C_1$-$C_2$-alkyl, benzyl or formyl;

(3) A compound of formula I, wherein

$R_4$, $R_5$, $R_6$, $R_7$, $R_8$, $R_9$, $R_{10}$, $R_{11}$, $R_{12}$ and $R_{13}$, independently of one another, are hydrogen, halogen, cyano, nitro, $C_1$-$C_6$-alkyl, halo-$C_1$-$C_6$-alkyl, $C_1$-$C_6$-alkoxy, halo-$C_1$-$C_6$-alkoxy, $C_3$-$C_6$-cycloalkyl, $C_1$-$C_6$-alkylthio, halo-$C_1$-$C_6$-alkylthio or, unsubstituted or one- to five-fold substituted aryl or unsubstituted or substituted hetaryl,

especially, independently of one another, hydrogen, halogen, $C_1$-$C_4$-alkyl, halo-$C_1$-$C_4$-alkyl, $C_1$-$C_4$-alkoxy or halo-$C_1$-$C_4$-alkoxy;

particularly, independently of one another, hydrogen, halogen, $C_1$-$C_2$-alkyl or halo-$C_1$-$C_2$-alkyl;

most preferably, independently of one another, hydrogen, halogen or $CF_3$;

(4) A compound of formula I, wherein

$X_1$ and $X_2$, independently of one another, are $N(R_{14})(R_{15})$, O or S;

especially, independently of one another, $NH_2$, O or S;

particularly O;

(5) A compound of formula I, wherein

$R_{14}$ and $R_{15}$, independently of one another, signify hydrogen, $C_1$-$C_4$-alkyl, formyl, $C_1$-$C_4$-alkylcarbonyl;

especially, independently of one another, hydrogen or $C_1$-$C_4$-alkyl;

particularly hydrogen;

(6) A compound of formula I, wherein

$R_1$ is hydrogen, halogen, $C_1$-$C_6$-alkyl, halo-$C_1$-$C_6$-alkyl, $C_1$-$C_6$-alkoxy, halo-$C_1$-$C_6$-alkoxy, $C_3$-$C_6$-cycloalkyl, halo-$C_3$-$C_6$-cycloalkyl, $C_3$-$C_6$-cycloalkyloxy, $C_3$-$C_6$-cycloalkylthio, $C_1$-$C_6$-alkylthio or halo-$C_1$-$C_6$-alkylthio;
$R_2$ and $R_3$, independently of one another, signify hydrogen, $C_1$-$C_6$-alkyl, formyl, $C_1$-$C_6$-alkylcarbonyl, $C_1$-$C_6$-alkoxycarbonyl, $C_1$-$C_6$-alkylaminocarbonyl, di-$C_1$-$C_6$-alkylaminocarbonyl or benzyl;
$R_4$, $R_5$, $R_6$, $R_7$, $R_8$, $R_9$, $R_{10}$, $R_{11}$, $R_{12}$ and $R_{13}$, independently of one another, are hydrogen, halogen, cyano, nitro, $C_1$-$C_6$-alkyl, halo-$C_1$-$C_6$-alkyl, $C_1$-$C_6$-alkoxy, halo-$C_1$-$C_6$-alkoxy, $C_3$-$C_6$-cycloalkyl, $C_1$-$C_6$-alkylthio, halo-$C_1$-$C_6$-alkylthio or unsubstituted or one- to five-fold substituted aryl or unsubstituted or substituted hetaryl,;
$X_1$ and $X_2$, independently of one another, are $N(R_{14})(R_{15})$, O or S; and
$R_{14}$ and $R_{15}$, independently of one another, signify hydrogen, $C_1$-$C_4$-alkyl, formyl, $C_1$-$C_4$-alkylcarbonyl;

(7) A compound of formula I, wherein

$R_1$ is hydrogen, halogen, $C_1$-$C_6$-alkyl, halo-$C_1$-$C_6$-alkyl, $C_1$-$C_6$-alkoxy or halo-$C_1$-$C_6$-alkoxy;
$R_2$ and $R_3$, independently of one another, signify hydrogen, $C_1$-$C_4$-alkyl, formyl, $C_1$-$C_4$-alkylcarbonyl or unsubstituted or one- to five-fold substituted benzyl;
$R_4$, $R_5$, $R_6$, $R_7$, $R_8$, $R_9$, $R_{10}$, $R_{11}$, $R_{12}$ and $R_{13}$, independently of one another, are hydrogen, halogen, $C_1$-$C_4$-alkyl, halo-$C_1$-$C_4$-alkyl, $C_1$-$C_4$-alkoxy or halo-$C_1$-$C_4$-alkoxy; and
$X_1$ and $X_2$, independently of one another, are $NH_2$, O or S;

(8) A compound of formula I, wherein

$R_1$ is hydrogen, $C_1$-$C_6$-alkyl or $C_1$-$C_6$-alkoxy;
$R_2$ and $R_3$, independently of one another, signify hydrogen, $C_1$-$C_2$-alkyl or formyl;
R4, $R_5$, $R_6$, $R_7$, $R_8$, $R_9$, $R_{10}$, $R_{11}$, $R_{12}$ and $R_{13}$, independently of one another, are hydrogen, halogen, $C_1$-$C_2$-alkyl or halo-$C_1$-$C_2$-alkyl; and
$X_1$ and $X_2$ are O;

(9) A compound of formula I, wherein

$R_1$ is hydrogen, $C_1$-$C_6$-alkyl or $C_1$-$C_6$-alkoxy;
$R_2$ and $R_3$, independently of one another, signify hydrogen, $C_1$-$C_2$-alkyl or formyl;
$R_4$, $R_5$, $R_6$, $R_7$, $R_8$, $R_9$, $R_{10}$, $R_{11}$, $R_{12}$ and $R_{13}$, independently of one another, are hydrogen, fluorine or $CF_3$; and
$X_1$ and $X_2$ are O.

Within the context of the invention, particular preference is given to the compounds of formula I listed in table 1, and most particularly those named in the synthesis examples.

The compounds of formula I of the present invention, in free form or in salt form respectively, may be prepared by a process for example characterised in that a compound of formula

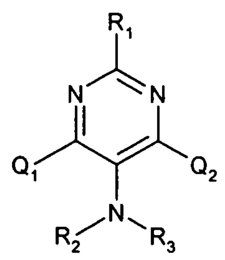

II,

which is known or may be produced analogously to corresponding known compounds, and wherein $R_1$, $R_2$ and $R_3$ are defined as given for formula I and $Q_1$ and $Q_2$ are leaving groups, is reacted with a compound of formula

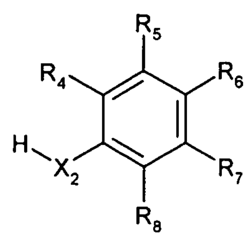

III,

which is known or may be produced analogously to corresponding known compounds, and wherein $R_4$, $R_5$, $R_6$, $R_7$, $R_8$ and $X_2$ are defined as given for formula I, and the intermediate is reacted subsequently or at the same time with a compound of formula

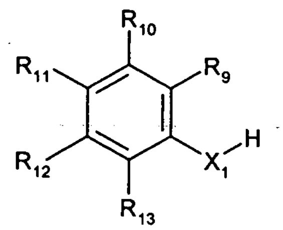

IV,

which is known or may be produced analogously to corresponding known compounds, and wherein $R_9$, $R_{10}$, $R_{11}$, $R_{12}$, $R_{13}$ and $X_1$ are defined as given for formula I,
and if desired, a compound of formula I obtainable according to the method or in another way, respectively in free form or in salt form, is converted into another compound of formula I, a mixture of isomers obtainable according to the method is separated and the desired isomer isolated and/or a free compound of formula I obtainable according to the method is converted into a salt or a salt of an compound of formula I obtainable according to the method is converted into the free compound of formula I or into another salt.

What has been stated above for salts of compounds I also applies analogously to salts of the starting materials listed hereinabove and hereinbelow.

The reaction partners can be reacted with one another as they are, i.e. without the addition of a solvent or diluent, e.g. in the melt. In most cases, however, the addition of an inert solvent or diluent, or a mixture thereof, is of advantage. Examples of such solvents or diluents are: aromatic, aliphatic and alicyclic hydrocarbons and halogenated hydrocarbons, such as benzene, toluene, xylene, mesitylene, tetraline, chlorobenzene, dichlorobenzene, bromobenzene, petroleum ether, hexane, cyclohexane, dichloromethane, trichloromethane, tetrachloromethane, dichloroethane, trichloroethene or tetrachloroethene; ethers, such as diethyl ether, dipropyl ether, diisopropyl ether, dibutyl ether, tert-butyl methyl ether, ethylene glycol monomethyl ether, ethylene glycol monoethyl ether, ethylene glycol dimethylether, dimethoxydiethylether, tetrahydrofuran or dioxane; ketones such as acetone, methyl ethyl ketone or methyl isobutyl ketone; amides such as N,N-dimethylformamide, N,N-diethylformamide, N,N-dimethylacetamide, N-methylpyrrolidone or hexamethylphosphoric acid triamide; nitriles such as acetonitrile or propionitrile; and sulfoxides, such as dimethyl sulfoxide. Preferred is N,N-dimethylformamide, N-methylpyrrolidone or tetrahydrofuran.

Preferred leaving groups Q are halogens, especially chlorine.

Suitable bases for facilitating the reaction are e.g. alkali metal or alkaline earth metal hydroxides, hydrides, amides, alkanolates, acetates, carbonates, dialkylamides or alkylsilylamides; alkylamines, alkylenediamines, optionally N-alkylated, optionally unsaturated, cycloalkylamines, basic heterocycles, ammonium hydroxides, as well as carbocyclic amines. Those which may be mentioned by way of example are sodium hydroxide, hydride, amide, methanolate, acetate, carbonate, potassium tert.-butanolate, hydroxide, carbonate, hydride, lithium diisopropylamide, potassium bis (trimethylsilyl)-amide, calcium hydride, triethylamine, diisopropylethylamine, triethylenediamine, cyclohexylamine, N-cyclohexyl-N,N-dimethylamine, N,N-diethylaniline, pyridine, 4-(N,N-dimethylamino)pyridine, quinuclidine, N-methylmorpholine, benzyltrimethylammonium hydroxide, as well as 1,5-diazabicyclo[5.4.0]undec-5-ene (DBU). Preferred is sodium hydride or potassium carbonate.

The reaction advantageously takes place in a temperature range of ca. 60°C to ca. 120°C , preferably from ca. 80°C to ca. 100°C .

Salts of compounds I may be produced in known manner. Acid addition salts, for example, are obtainable from compounds I by treating with a suitable acid or a suitable ion exchange reagent, and salts with bases are obtainable by treating with a suitable base or a suitable ion exchange reagent

Salts of compounds I can be converted into the free compounds I by the usual means, acid addition salts e. g. by treating with a suitable basic composition or with a suitable ion exchange reagent, and salts with bases e.g. by treating with a suitable acid or a suitable ion exchange reagent.

Salts of compounds I can be converted into other salts of compounds I in a known manner; acid addition salts can be converted for example into other acid addition salts, e.g. by treating a salt of an inorganic acid, such as a hydrochloride, with a suitable metal salt, such as a sodium, barium, or silver salt, of an acid, e.g. with silver acetate, in a suitable solvent, in which a resulting inorganic salt, e.g. silver chloride, is insoluble and thus precipitates out from the reaction mixture.

Depending on the method and/or reaction conditions, compounds I with salt-forming characteristics can be obtained in free form or in the form of salts.

Compounds I can also be obtained in the form of their hydrates and/or also can include other solvents, used for example where necessary for the crystallisation of compounds present in solid form.

The compounds I may be optionally present as optical and/or geometric isomers or as a mixture thereof. The invention relates both to the pure isomers and to all possible isomeric mixtures, and is hereinbefore and hereinafter

understood as doing so, even if stereochemical details are not specifically mentioned in every case.

Diastereoisomeric mixtures of compounds I, which are obtainable by the process or in another way, may be separated in known manner, on the basis of the physical-chemical differences in their components, into the pure diastereoisomers, for example by fractional crystallisation, distillation and/or chromatography.

Splitting of mixtures of enantiomers that are obtainable accordingly may be achieved by known methods, for example by recrystallisation from an optically active solvent, by chromatography on chiral adsorbents, e.g. high-pressure liquid chromatography (HPLC) on acetyl cellulose, with the assistance of appropriate micro-organisms, by cleavage with specific immobilised enzymes, through the formation of inclusion compounds, e.g. using chiral crown ethers, whereby only one enantiomer is complexed.

According to the invention, apart from separation of corresponding isomer mixtures, generally known methods of diastereoselective or enantioselective synthesis can also be applied to obtain pure diastereoisomers or enantiomers, e.g. by carrying out the method of the invention using educts with correspondingly suitable stereochemistry.

It is advantageous to isolate or synthesise the biologically more active isomer, e.g. enantiomer, provided that the individual components have differing biological efficacy.

In the method of the present invention, the starting materials and intermediates used are preferably those that lead to the compounds I described at the beginning as being especially useful.

The invention relates in particular to the preparation methods described in the examples.

Starting materials and intermediates, which are new and are used according to the invention for the preparation of compounds I, as well as their usage and process for the preparation thereof, similarly form an object of the invention.

The compounds of the formula I according to the invention are notable for their broad activity spectrum and are valuable active ingredients for use in pest control. They are particularly suitable in the control of ectoparasites and to a certain extent also for controlling endoparasites on and in animals and in the hygiene field, whilst being well tolerated by warm-blooded animals.

In the context of the present invention, ectoparasites are understood to be in particular insects, acari (mites and ticks), and crustaceans (sea lice). These include insects of the following orders: *Lepidoptera, Coleoptera, Homoptera, Hemiptera, Heteroptera, Diptera, Dictyoptera, Thysanoptera, Orthoptera, Anoplura, Siphonaptera, Mallophaga, Thysanura, Isoptera, Psocoptera* and *Hymenoptera.* However, the ectoparasites which may be mentioned in particular are those which trouble humans or animals and carry pathogens, for example flies such as *Musca domestica, Musca vetustissima, Musca autumnalis, Fannia canicularis, Sarcophaga camaria, Lucilia cuprina, Lucilia sericata, Hypoderma bovis, Hypoderma lineatum, Chrysomyia chloropyga, Dermatobia hominis, Cochliomyia hominivorax, Gasterophilus intestinalis, Oestrus ovis, Stomoxys calcitrans, Haematobia irritans* and biting insects like midges, such as *Ceratopogonidae* (biting midges), *Simuliidae* (Blackflies), *Psychodidae* (Sandflies); but also blood-sucking parasites, for example fleas, such as *Ctenocephalides felis* and *Ctenocephalides canis* (cat and dog fleas), *Xenopsylla cheopis, Pulex irritans, Ceratophylllus gallinae, Dermatophilus penetrans;* lice, such as *Damalina ovis, Pediculus humanis*; mosquitoes, such as *Anopheles* spp, *Aedes* spp, *Culex* spp.; biting flies such as *Haematobia irritans, Stomoxys calcitrans;* horse-flies (Tabanids) with the subfamilies of Tabanidae such as *Haematopota spp. (e.g. Haematopota pluvialis)* and *Tabanus spp, (*e.g. *Tabanus nigrovittatus)* and *Chrysopsinae* such as *Chrysops spp. (e.g. Chrysops caecutiens);* tsetse flies, such as species of *Glossinia*. Ectoparasites also include members of the order Acarina, such as mites (e.g. *Chorioptes bovis, Cheyletiella* spp., *Dermanyssus gallinae, Demodex canis, Sarcoptes scabiei, Psoroptes ovis* and *Psorergates spp.* and ticks. Known representatives of ticks are, for example, *Boophilus, Amblyomma, Anocentor, Dermacentor, Haemaphysalis, Hyalomma, Ixodes, Rhipicentor, Margaropus, Rhipicephalus, Argas, Otobius* and *Omithodoros* and the like, which preferably infest warm-blooded animals including farm animals, such as cattle, horses, pigs, sheep and goats, poultry such as chickens, turkeys and geese, fur-bearing animals such as mink, foxes, chinchillas, rabbits and the like, as well as domestic animals such as cats and dogs, but also humans.

The compounds of the formula I according to the invention are also active against all or individual development stages of animal pests showing normal sensitivity, as well as those showing resistance to widely used parasiticides. This is especially true for resistant insects and members of the order *Acarina*. The insecticidal, ovicidal and/or acaricidal effect of the active substances of the invention can manifest itself directly, i.e. killing the pests either immediately or after some time has elapsed, for example when moulting occurs, or by destroying their eggs, or indirectly, e.g. reducing the number of eggs laid and/or the hatching rate, good efficacy corresponding to a pesticidal rate (mortality) of at least 50 to 60%.

Compounds of the formula I can also be used against hygiene pests, especially of the order *Diptera* of the families *Muscidae, Sarcophagidae, Anophilidae* and *Culicidae;* the orders *Orthoptera, Dictyoptera* (e.g. the family *Blattidae* (cockroaches), such as *Blatella germanica, Blatta orientalis, Periplaneta americana)* and *Hymenoptera* (e.g. the families *Formicidae* (ants) and *Vespidae* (wasps).

Surprisingly, the compounds of formula I are also effective against ectoparasites of fishes, especially the subclass of Copepoda (e.g. order of *Siphonostognatoidae* (sea lice), whilst being well tolerated by fish.

Certain compounds of the formula I seem to be also effective against ertain species of helminths.

Helminths are commercially important because they cause serious diseases in mammals and poultry, e.g. in sheep, pigs, goats, cattle, horses, donkeys, dogs, cats, guinea-pigs and exotic birds. Typical nematodes are: *Haemonchus, Trichostrongylus, Ostertagia, Nematodirus, Cooperia, Ascaris, Bunostonum, Oesophagostonum, Charbertia, Trichuris, Strongylus, Trichonema, Dictyocaulus, Capillaria, Heterakis, Toxocara, Ascaridia, Oxyuris, Ancylostoma, Uncinaria, Toxascaris* and *Parascaris*. The trematodes include, in particular, the family of *Fasciolideae,* especially *Fasciola hepatica*.

The good pesticidal activity of the compounds of formula I according to the invention corresponds to a mortality rate of at least 50-60% of the pests mentioned, more preferably to a mortality rate over 90%, most preferably to 95-100%. The compounds of formula I are preferably employed internally and externally in unmodified form or preferably together with the adjuvants conventionally used in the art of formulation and may therefore be processed in a known manner to give, for example, liquid formulations (e.g. spot-on, pour-on, spray-on, emulsions, suspensions, solutions, emulsifiable concentrates, solution concentrates), semi-solid formulations (e.g.creams, ointments, pastes, gels, liposomal preparations) and solid preparations (e.g. food additives tablets including e, capsules,, powders including soluble powders, granules, embeddings of the active ingredient in polymeric substances, like implants and microparticles). As with the compositions, the methods of application are selected in accordance with the intended objectives and the prevailing circumstances.

The formulation, i.e. preparations containing the active ingredient of formula I, or combinations of these active ingredients with other active ingredients, and optionally a solid, semi-solid or liquid adjuvant, are produced in a manner known *per se*, for example by intimately mixing, kneading or dispersing the active ingredients with compositions of excipients, whereby the physiological compatibility of the formulation excipients must be taken into consideration.

The solvents in question may be: alcohols (aliphatic and aromatic), such as benzylalcohol, ethanol, propanol, isopropanol or butanol, fatty alcohols, such as oleyl alcohol and glycols and their ethers and esters, such as glycerin, propylene glycol, dipropylene glycol ether, ethylene glycol, ethylene glycol monomethyl or -ethyl ether and butyl dioxytol, ketones, such as propylene carbonate, cyclohexanone, isophorone or diacetanol alcohol and polyethylene glycols, such as PEG 300. In addition, the compositions may comprise strong polar solvents, such as N-methyl-2-pyrrolidone, dimethyl sulfoxide or dimethylformamide, or water, fatty acid esters, such as ethyl oleate or isopropylpalmitate, vegetable oils, such as rape, castor, coconut, or soybean oil, synthetic mono-, di-, triglycerides like e.g. glyceryl monostearate and medium chain triglycerides and also, if appropriate, silicone oils. The mentioned ingredients may also serve as carrier for particulate application froms.

As ointment base resp. structure building ingredients the following excipients may be used: Petroleum based substances, such as Vaseline or paraffines, bases made from wool fat, like e.g. lanolin or lanolin alcohols, polyethylene glycols like e.g. macrogols and lipid bases like e.g. phospholipids or triglycerids, such as hydrogenated vegetable oils.

The use of emulsifiers, wetting agents and spreading agents may also be required, in general, lecithins like soy lecithin, salts of fatty acids with alkaline earth and alkali metals, alkyl sulfates like sodium cetylstearyl sulphate, cholates, fatty alcohols like cetyl alcohol, sterols like cholestesterol, polyoxyethylene sorbitan fatty acid esters like polysorbate 20, sorbitan fatty acid esters like sorbitan mono laureate, fatty acid esters and fatty alcohol ethers of polyoxyethylene like poloxyl oleyl ether, polyoxypropylene polyoxyethylene block copolymers as e.g. Pluronic™ , saccharose esters like saccharose distearate, polyglyceryl fatty acid esters like polyglycerol oleate and fatty acid esters like e.g. ethyl oleate or isopropylmyristate

The formulations may also include gelifying and stiffening agents, like e.g. polyacrylic acid derivatives, cellulose ethers, polyvinyl alcohols, polyvinylpyrrolidons and fine disperse silicium dioxide.

As polymeric agents with controlled release properties, may be applied derivatives made by e.g. polylactic acid, polylactic coglycolic acid, poly orthoester, polyethylene carbonate, poly anhydrids and starch and PVC based matrices

The addition of penetration enhancers like ketons, sulfoxids, amids, fatty acid esters and fatty alcohols may be necessary.

Also preservatives like sorbic acid, benzyl alcohol and parabenes, and antioxidants as e.g. alpha tocopherol may be added.

The active ingredient or combinations of the active ingredient may also applied in capsules, like hard gelatine capsules or soft capsules.

The binders for tablets and boli may be chemically modified polymeric natural substances that are soluble in water or in alcohol, such as starch, cellulose or protein derivatives (e.g. methyl cellulose, carboxymethyl cellulose, ethylhydroxyethyl cellulose, proteins such as zein, gelatin and the like), as well as synthetic polymers, such as polyvinyl alcohol, polyvinyl pyrrolidone etc. The tablets also contain fillers (e.g. starch, microcrystalline cellulose, sugar, lactose etc.), glidants (e.g. magnesium stearate) and disintegrants (e.g. cellulose derivatives) and acid resistant coatings, like e.g. acrylic acid esters.

The compounds of formula I according to the invention may be used alone or in combination with other biocides. They may be combined with pesticides having the same sphere of activity e.g. to increase activity, or with substances having another sphere of activity e.g. to broaden the range of activity. It can also be sensible to add so-called repellents. Since the compounds of formula I are adulticides, i.e. since they are effective in particular against the adult stage of the target parasites, the addition of pesticides which instead attack the juvenile stages of the parasites may be very advantageous. In this way, the greatest part of those parasites that produce great economic damage will be covered. Moreover, this action will contribute substantially to avoiding the formation of resistance. Many combinations may also lead to synergistic effects, i.e. the total amount of active ingredient can be reduced, which is desirable from an ecological point of view. Preferred groups of combination partners and especially preferred combination partners are named in the following, whereby combinations may contain one or more of these partners in addition to a compound of formula I.

Suitable partners in the mixture may be biocides, e.g. the insecticides and acaricides with a varying mechanism of activity, which are named in the following and have been known to the person skilled in the art for a long time, e.g. chitin synthesis inhibitors, growth regulators; active ingredients which act as juvenile hormones; active ingredients which act as adulticides; broad-band insecticides, broad-band acaricides and nematicides; and also the well known anthelminthics and insect- and/or acarid-deterring substances, said repellents or detachers.

Non-limitative examples of suitable insecticides and acaricides are:

| | | |
|---|---|---|
| 1. Abamectin | 13. Azinphos M | 25. Bufencarb |
| 2. AC 303 630 | 14. Azinphos-methyl | 26. Buprofezin |
| 3. Acephat | 15. Azocyclotin | 27. Butocarboxin |
| 4. Acrinathrin | 16. *Bacillus subtil.* toxin | 28. Butylpyridaben |
| 5. Alanycarb | 17. Bendiocarb | 29. Cadusafos |
| 6. Aldicarb | 18. Benfuracarb | 30. Carbaryl |
| 7. α-Cypermethrin | 19. Bensultap | 31. Carbofuran |
| 8. Alphamethrin | 20. β-Cyfluthrin | 32. Carbophenthion |
| 9. Amitraz | 21. Bifenthrin | 33. Cartap |
| 10. Avermectin $B_1$ | 22. BPMC | 34. Chloethocarb |
| 11. AZ 60541 | 23. Brofenprox | 35. Chlorethoxyfos |
| 12. Azinphos A | 24. Bromophos A | 36. Chlorfenapyr |

| |
|---|
| 37. Chlorfluazuron |
| 38. Chlormephos |
| 39. Chlorpyrifos |
| 40. Cis-Resmethrin |
| 41. Clocythrin |
| 42. Clofentezin |
| 43. Cyanophos |
| 44. Cycloprothrin |
| 45. Cyfluthrin |
| 46. Cyhexatin |
| 47. D 2341 |
| 48. Deltamethrin |
| 49. Demeton M |
| 50. Demeton S |
| 51. Demeton-S-methyl |
| 52. Dibutylaminothio |
| 53. Dichlofenthion |
| 54. Dicliphos |
| 55. Diethion |
| 56. Diflubenzuron |
| 57. Dimethoat |
| 58. Dimethylvinphos |
| 59. Dioxathion |
| 60. DPX-MP062 |
| 61. Edifenphos |
| 62. Emamectin |
| 63. Endosulfan |
| 64. Esfenvalerat |
| 65. Ethiofencarb |
| 66. Ethion |
| 67. Ethofenprox |
| 68. Ethoprophos |
| 69. Etrimphos |

| |
|---|
| 70. Fenamiphos |
| 71. Fenazaquin |
| 72. Fenbutatinoxid |
| 73. Fenitrothion |
| 74. Fenobucarb |
| 75. Fenothiocarb |
| 76. Fenoxycarb |
| 77. Fenpropathrin |
| 78. Fenpyrad |
| 79. Fenpyroximate |
| 80. Fenthion |
| 81. Fenvalerate |
| 82. Fipronil |
| 83. Fluazinam |
| 84. Fluazuron |
| 85. Flucycloxuron |
| 86. Flucythrinat |
| 87. Flufenoxuron |
| 88. Flufenprox |
| 89. Fonophos |
| 90. Formothion |
| 91. Fosthiazat |
| 92. Fubfenprox |
| 93. HCH |
| 94. Heptenophos |
| 95. Hexaflumuron |
| 96. Hexythiazox |
| 97. Hydroprene |
| 98. Imidacloprid |
| 99. insect-active fungi |
| 100. insect-active nematodes |

| |
|---|
| 101. insect-active viruses |
| 102. Iprobenfos |
| 103. Isofenphos |
| 104. Isoprocarb |
| 105. Isoxathion |
| 106. Ivermectin |
| 107. λ-Cyhalothrin |
| 108. Lufenuron |
| 109. Malathion |
| 110. Mecarbam |
| 111. Mesulfenphos |
| 112. Metaldehyd |
| 113. Methamidophos |
| 114. Methiocarb |
| 115. Methomyl |
| 116. Methoprene |
| 117. Metolcarb |
| 118. Mevinphos |
| 119. Milbemectin |
| 120. Moxidectin |
| 121. Naled |
| 122. NC 184 |
| 123. NI-25, Acetamiprid |
| 124. Nitenpyram |
| 125. Omethoat |
| 126. Oxamyl |
| 127. Oxydemethon M |
| 128. Oxydeprofos |
| 129. Parathion |
| 130. Parathion-methyl |
| 131. Permethrin |
| 132. Phenthoat |

| | | |
|---|---|---|
| 133. Phorat | 152. RH 5992 | 171. Thiofanox |
| 134. Phosalone | 153. RH-2485 | 172. Thionazin |
| 135. Phosmet | 154. Salithion | 173. Thuringiensin |
| 136. Phoxim | 155. Sebufos | 174. Tralomethrin |
| 137. Pirimicarb | 156. Silafluofen | 175. Triarthen |
| 138. Pirimiphos A | 157. Spinosad | 176. Triazamate |
| 139. Pirimiphos M | 158. Sulfotep | 177. Triazophos |
| 140. Promecarb | 159. Sulprofos | 178. Triazuron |
| 141. Propaphos | 160. Tebufenozide | 179. Trichlorfon |
| 142. Propoxur | 161. Tebufenpyrad | 180. Triflumuron |
| 143. Prothiofos | 162. Tebupirimphos | 181. Trimethacarb |
| 144. Prothoat | 163. Teflubenzuron | 182. Vamidothion |
| 145. Pyrachlophos | 164. Tefluthrin | 183. XMC (3,5,-Xylyl-methylcarbamate) |
| 146. Pyradaphenthion | 165. Temephos | |
| 147. Pyresmethrin | 166. Terbam | 184. Xylylcarb |
| 148. Pyrethrum | 167. Terbufos | 185. YI 5301/5302 |
| 149. Pyridaben | 168. Tetrachlorvinphos | 186. ζ-Cypermethrin |
| 150. Pyrimidifen | 169. Thiafenox | 187. Zetamethrin |
| 151. Pyriproxyfen | 170. Thiodicarb | |

Non-limitative examples of suitable anthelminthics are named in the following, a few representatives have anthelminthic activity in addition to the insecticidal and acaricidal activity, and are partly already in the above list.

(A1) Praziguantel = 2-cyclohexylcarbonyl-4-oxo-1,2,3,6,7,11b-hexahydro-4H-pyrazino[2,1-α]isoquinoline
(A2) Closantel = 3,5-diiodo-N-[5-chloro-2-methyl-4-(a-cyano-4-chlorobenzyl)phenyl]-salicylamide
(A3) Triclabendazole = 5-chloro-6-(2,3-dichlorophenoxy)-2-methylthio-1H-benzimidazole
(A4) Levamisol = *L*-(-)-2,3,5,6-tetrahydro-6-phenylimidazo[2,1b]thiazole
(A5) Mebendazole = (5-benzoyl-1H-benzimidazol-2-yl)carbaminic acid methylester
(A6) Omphalotin = a macrocyclic fermentation product of the fungus *Omphalotus olearius* described in WO 97/20857
(A7) Abamectin = avermectin B1
(A8) Ivermectin = 22,23-dihydroavermectin B1
(A9) Moxidectin = 5-O-demethyl-28-deoxy-25-(1,3-dimethyl-1-butenyl)-6,28- epoxy-23-(methoxyimino)-milbemycin B
(A10) Doramectin = 25-cyclohexyl-5-O-demethyl-25-de(1-methylpropyl)-avermectin A1a
(A11) Milbemectin = mixture of milbemycin A3 and milbemycin A4
(A12) Milbemycinoxim = 5-oxime of milbemectin

Non-limitative examples of suitable repellents and detachers are:

(R1) DEET (N, N-diethyl-m-toluamide)
(R2) KBR 3023 N-butyl-2-oxycarbonyl-(2-hydroxy)-piperidine
(R3) Cymiazole = N,-2,3-dihydro-3-methyl-1,3-thiazol-2-ylidene-2,4-xylidene

The said partners in the mixture are best known to specialists in this field. Most are described in various

editions of the Pesticide Manual, The British Crop Protection Council, London, and others in the various editions of The Merck Index, Merck & Co., Inc., Rahway, New Jersey, USA or in patent literature. Therefore, the following listing is restricted to a few places where they may be found by way of example.

(I) 2-Methyl-2-(methylthio)propionaldehyde-*O*-methylcarbamoyloxime (Aldicarb), from The Pesticide Manual, 11$^{th}$ Ed. (1997), The British Crop Protection Council, London, page 26;
(II) *S*-(3,4-dihydro-4-oxobenzo[*d*]-[1,2,3]-triazin-3-ylmethyl)O,O-dimethyl-phosphorodithioate (Azinphos-methyl), from The Pesticide Manual, 11$^{th}$Ed. (1997), The British Crop Protection Council, London, page 67;
(III) Ethyl-N-[2,3-dihydro-2,2-dimethylbenzofuran-7-yloxycarbonyl-(methyl)aminothio]-N-isopropyl-β-alaninate (Benfuracarb), from The Pesticide Manual, 11$^{th}$Ed. (1997), The British Crop Protection Council, London, page 96;
(IV) 2-Methylbiphenyl-3-ylmethyl-(*Z*)-(1*RS*)-*cis*-3-(2-chloro-3,3,3-trifluoroprop-1-enyl)-2,2-dimethylcyclopropanecarboxylate (Bifenthrin), from The Pesticide Manual, 11 $^{th}$Ed. (1997), The British Crop Protection Council, London, page 118;
(V) 2-tert-butylimino-3-isopropyl-5-phenyl-1,3,5-thiadiazian-4-one (Buprofezin), from The Pesticide Manual, 11$^{th}$Ed. (1997), The British Crop Protection Council, London, page 157;
(VI) 2,3-Dihydro-2,2-dimethylbenzofuran-7-yl-methylcarbamate (Carbofuran), from The Pesticide Manual, 11$^{th}$Ed. (1997), The British Crop Protection Council, London, page 186;
(VII) 2,3-Dihydro-2,2-dimethylbenzofuran-7-yl-(dibutylaminothio)methylcarbamate (Carbosulfan), from The Pesticide Manual, 11$^{th}$Ed. (1997), The British Crop Protection Council, London, page 188;
(VIII) *S*,*S*'-(2-dimethylaminotrimethylene)-bis(thiocarbamate) (Cartap), from The Pesticide Manual, 11$^{th}$Ed. (1997), The British Crop Protection Council, London, page 193;
(IX) 1-[3,5-Dichloro-4-(3-chloro-5-trifluoromethyl-2-pyridyloxy)phenyl]-3-(2,6-difluorobenzoyl)-urea (Chlorfluazuron), from The Pesticide Manual, 11$^{th}$Ed. (1997), The British Crop Protection Council, London, page 213;
(X) *O*,*O*-diethyl-*O*-3,5,6-trichloro-2-pyridyl-phosphorothioate (Chlorpyrifos), from The Pesticide Manual, 11$^{th}$Ed. (1997), The British Crop Protection Council, London, page 235;
(XI) (*RS*)-α-cyano-4-fluoro-3-phenoxybenzyl-(1*RS*,3*RS*;1*RS*,3*RS*)-3-(2,2-dichlorovinyl)-2,2-di-methylcyclopropanecarboxylate (Cyfluthrin), from The Pesticide Manual, 11$^{th}$Ed. (1997), The British Crop Protection Council, London, page 293;
(XII) Mixture of (*S*)-α-cyano-3-phenoxybenzy)-(Z)-(1*R*,3*R*)-3-(2-chloro-3,3,3-trifluoropropenyl)-2,2-dimethylcyclopropanecarboxylate and (*R*)-α-cyano-3-phenoxybenzyl-(*Z*)-(1*R*,3*R*)-3-(2-chloro-3,3,3-trifluoropropenyl)-2,2-dimethylcyclopropanecarboxylate (Lambda-Cyhalothrin), from The Pesticide Manual, 11$^{th}$Ed. (1997), The British Crop Protection Council, London, page 300;
(XIII) Racemate consisting of (*S*)-α-cyano-3-phenoxybenzyl-(*Z*)-(1*R*,3*R*)-3-(2,2-dichlorovinyl)-2,2-dimethylcyclopropanecarboxylate and (*R*)-α-cyano-3-phenoxybenzyl-(1 *S*,3*S*)-3-(2,2-dichlorovinyl)-2,2-dimethylcyclopropanecarboxylate (Alpha-cypermethrin), from The Pesticide Manual, 11$^{th}$Ed. (1997), The British Crop Protection Council, London, page 308;
(XIV) a mixture of the stereoisomers of (*S*)-α-cyano-3-phenoxybenzyl (1*RS*,3*RS*,-1*RS*,3*RS*)-3-(2,2-dichlorovinyl)-2,2-dimethylcyclopropanecarboxylate (zeta-Cypermethrin), from The Pesticide Manual, 11$^{th}$Ed. (1997), The British Crop Protection Council, London, page 314;
(XV) (*S*)-α-cyano-3-phenoxybenzyl-(1*R*,3*R*)-3-(2,2-dibromovinyl)-2,2-dimethylcyclopropanecarboxylate (Deltamethrin), from The Pesticide Manual, 11$^{th}$Ed. (1997), The British Crop Protection Council, London, page 344;
(XVI) (4-chlorophenyl)-3-(2,6-difluorobenzoyl)urea (Diflubenzuron), from The Pesticide Manual, 11$^{th}$Ed. (1997), The British Crop Protection Council, London, page 395;
(XVII) (1,4,5,6,7,7-Hexachloro-8,9,10-trinorborn-5-en-2,3-ylenebismethylene)-sulphite (Endosulfan), from The Pesticide Manual, 11$^{th}$Ed. (1997), The British Crop Protection Council, London, page 459;
(XVIII) α-ethylthio-o-tolyl-methylcarbamate (Ethiofencarb), from The Pesticide Manual, 11$^{th}$Ed. (1997), The British Crop Protection Council, London, page 479;
(XIX) *O*,*O*-dimethyl-*O*-4-nitro-*m*-tolyl-phosphorothioate (Fenitrothion), from The Pesticide Manual, 11$^{th}$Ed. (1997), The British Crop Protection Council, London, page 514;
(XX) 2-sec-butylphenyl-methylcarbamate (Fenobucarb), from The Pesticide Manual, 11$^{th}$Ed. (1997), The British Crop Protection Council, London, page 516;
(XXI) (*RS*)-α-cyano-3-phenoxybenzyl-(*RS*)-2-(4-chlorophenyl)-3-methylbutyrate (Fenvalerate), from The Pesticide Manual, 11$^{th}$Ed. (1997), The British Crop Protection Council, London, page 539;
(XXII) *S*-[formyl(methyl)carbamoylmethyl]-*O*,*O*-dimethyl-phosphorodithioate (Formothion), from The Pesticide Manual, 11$^{th}$Ed. (1997), The British Crop Protection Council, London, page 625;
(XXIII) 4-Methylthio-3,5-xylyl-methylcarbamate (Methiocarb), from The Pesticide Manual, 11$^{th}$Ed. (1997), The British Crop Protection Council, London, page 813;
(XXIV) 7-Chlorobicyclo[3.2.0]hepta-2,6-dien-6-yl-dimethylphosphate (Heptenophos), from The Pesticide Manual,

11thEd. (1997), The British Crop Protection Council, London, page 670;
(XXV) 1-(6-chloro-3-pyridylmethyl)-N-nitroimidazolidin-2-ylidenamine (Imidacloprid), from The Pesticide Manual, 11thEd. (1997), The British Crop Protection Council, London, page 706;
(XXVI) 2-isopropylphenyl-methylcarbamate (Isoprocarb), from The Pesticide Manual, 11thEd. (1997), The British Crop Protection Council, London, page 729;
(XXVII) *O,S*-dimethyl-phosphoramidothioate (Methamidophos), from The Pesticide Manual, 11thEd. (1997), The British Crop Protection Council, London, page 808;
(XXVIII) S-Methyl-N-(methylcarbamoyloxy)thioacetimidate (Methomyl), from The Pesticide Manual, 11thEd. (1997), The British Crop Protection Council, London, page 815;
(XXIX) Methyl-3-(dimethoxyphosphinoyloxy)but-2-enoate (Mevinphos), from The Pesticide Manual, 11thEd. (1997), The British Crop Protection Council, London, page 844;
(XXX) *O,O*-diethyl-*O*-4-nitrophenyl-phosphorothioate (Parathion), from The Pesticide Manual, 11thEd. (1997), The British Crop Protection Council, London, page 926;
(XXXI) *O,O*-dimethyl-*O*-4-nitrophenyl-phosphorothioate (Parathion-methyl), from The Pesticide Manual, 11thEd. (1997), The British Crop Protection Council, London, page 928;
(XXXII) *S*-6-chloro-2,3-dihydro-2-oxo-1,3-benzoxazol-3-ylmethyl-*O*,*O*-diethyl-phosphordithioate (Phosalone), from The Pesticide Manual, 11thEd. (1997), The British Crop Protection Council, London, page 963;
(XXXIII) 2-Dimethylamino-5,6-dimethylpyrimidin-4-yl-dimethylcarbamate (Pirimicarb), from The Pesticide Manual, 11thEd. (1997), The British Crop Protection Council, London, page 985;
(XXXIV) 2-isopropoxyphenyl-methylcarbamate (Propoxur), from The Pesticide Manual, 11thEd. (1997), The British Crop Protection Council, London, page 1036;
(XXXV) 1-(3,5-dichloro-2,4-difluorophenyl)-3-(2,6-difluorobenzoyl)urea (Teflubenzuron), from The Pesticide Manual, 11thEd. (1997), The British Crop Protection Council, London, page 1158;
(XXXVI) *S*-tert-butylthiomethyl-*O,O*-dimethyl-phosphorodithioate (Terbufos), from The Pesticide Manual, 11thEd. (1997), The British Crop Protection Council, London, page 1165;
(XXXVII) ethyl-(3-*tert*.-butyl-1-dimethylcarbamoyl-1*H*-1,2,4-triazol-5-yl-thio)-acetate, (Triazamate), from The Pesticide Manual, 11thEd. (1997), The British Crop Protection Council, London, page 1224;
(XXXVIII) Abamectin, from The Pesticide Manual, 11thEd. (1997), The British Crop Protection Council, London, page 3;
(XXXIX) 2-sec-butylphenyl-methylcarbamate (Fenobucarb), from The Pesticide Manual, 11thEd. (1997), The British Crop Protection Council, London, page 516;
(XL) *N-tert*.-butyl-*N'*-(4-ethylbenzoyl)-3,5-dimethylbenzohydrazide (Tebufenozide), from The Pesticide Manual, 11thEd. (1997), The British Crop Protection Council, London, page 1147;
(XLI) (±)-5-amino-1-(2,6-dichloro-α,α,α-trifluoro-p-tolyl)-4-trifluoromethyl-sulphinylpyrazol-3-carbonitrile (Fipronil), from The Pesticide Manual, 11thEd. (1997), The British Crop Protection Council, London, page 545;
(XLII) (*RS*)-α-cyano-4-fluoro-3-phenoxybenzyl(1*RS*,3*RS*;1*RS*,3*RS*)-3-(2,2-dichlorovinyl)-2,2-dimethylcyclopropanecarboxylate (beta-Cyfluthrin), from The Pesticide Manual, 11thEd. (1997), The British Crop Protection Council, London, page 295;
(XLIII) (4-ethoxyphenyl)-[3-(4-fluoro-3-phenoxyphenyl)propyl](dimethyl)silane (Silafluofen), from The Pesticide Manual, 11thEd. (1997), The British Crop Protection Council, London, page 1105;
(XLIV) *tert*.-butyl (*E*)-α-(1,3-dimethyl-5-phenoxypyrazol-4-yl-methylenamino-oxy)-p-toluate (Fenpyroximate), from The Pesticide Manual, 11thEd. (1997), The British Crop Protection Council, London, page 530;
(XLV) 2-*tert*.-butyl-5-(4-*tert*.-butylbenzylthio)-4-chloropyridazin-3(2*H*)-one (Pyridaben), from The Pesticide Manual, 11thEd. (1997), The British Crop Protection Council, London, page 1161;
(XLVI) 4-[[4-(1,1-dimethylphenyl)phenyl]ethoxy]-quinazoline (Fenazaquin), from The Pesticide Manual, 11thEd. (1997), The British Crop Protection Council, London, page 507;
(XLVII) 4-phenoxyphenyl-(*RS*)-2-(pyridyloxy)propyl-ether (Pyriproxyfen), from The Pesticide Manual, 11thEd. (1997), The British Crop Protection Council, London, page 1073;
(XLVIII) 5-chloro-*N*-{2-[4-(2-ethoxyethyl)-2,3-dimethylphenoxy]ethyl}-6-ethylpyrimidine-4-amine (Pyrimidifen), from The Pesticide Manual, 11thEd. (1997), The British Crop Protection Council, London, page 1070;
(XLIX) (*E*)-*N*-(6-chloro-3-pyridylmethyl)-*N*-ethyl-*N'*-methyl-2-nitrovinylidenediamine (Nitenpyram), from The Pesticide Manual, 11thEd. (1997), The British Crop Protection Council, London, page 880;
(L) (*E*)-$N^1$-[(6-chloro-3-pyridyl)methyl]-$N^2$-cyano-$N^1$-methylacetamidine (NI-25, Acetamiprid), from The Pesticide Manual, 11thEd. (1997), The British Crop Protection Council, London, page 9;
(LI) Avermectin $B_1$ , from The Pesticide Manual, 11thEd. (1997), The British Crop Protection Council, London, page 3;
(LII) an insect-active extract from a plant, especially (2*R*,6*aS*,12*aS*)-1,2,6,6a,12,12a-hexhydro-2-isopropenyl-8,9-dimethoxy-chromeno[3,4-*b*]furo[2,3-*h*]chromen-6-one (Rotenone), from The Pesticide Manual, 11thEd. (1997),

The British Crop Protection Council, London, page 1097; and an extract from *Azadirachta indica*, especially azadirachtin, from The Pesticide Manual, 11thEd. (1997), The British Crop Protection Council, London, page 59; and
(LIII) a preparation which contains insect-active nematodes, preferably *Heterorhabditis bacteriophora* and *Heterorhabditis megidis,* from The Pesticide Manual, 11thEd. (1997), The British Crop Protection Council, London, page 671; *Steinernema feltiae*, from The Pesticide Manual, 11thEd. (1997), The British Crop Protection Council, London, page 1115 and *Steinernema scapterisci*, from The Pesticide Manual, 11thEd. (1997), The British Crop Protection Council, London, page 1116;
(LIV) a preparation obtainable from *Bacillus subtilis*, from The Pesticide Manual, 11thEd. (1997), The British Crop Protection Council, London, page 72; or from a strain of *Bacillus thuringiensis* with the exception of compounds isolated from GC91 or from NCTC11821; The Pesticide Manual, 11thEd. (1997), The British Crop Protection Council, London, page 73;
(LV) a preparation which contains insect-active fungi, preferably *Verticillium lecanii*, from The Pesticide Manual, 11thEd. (1997), The British Crop Protection Council, London, page *1266; Beauveria brogniartii*, from The Pesticide Manual, 11thEd. (1997), The British Crop Protection Council, London, page 85 and *Beauveria bassiana*, from The Pesticide Manual, 11thEd. (1997), The British Crop Protection Council, London, page 83;
(LVI) a preparation which contains insect-active viruses, preferably *Neodipridon Sertifer NPV,* from The Pesticide Manual, 11thEd. (1997), The British Crop Protection Council, London, page 1342; *Mamestra brassicae* NPV, from The Pesticide Manual, 11thEd. (1997), The British Crop Protection Council, London, page 759 and *Cydia pomonella granulosis* virus, from The Pesticide Manual, 11thEd. (1997), The British Crop Protection Council, London, page 291;
(CLXXXI) 7-chloro-2,3,4*a*,5-tetrahydro-2-[methoxycarbonyl(4-trifluoromethoxyphenyl)-carbamoyl]indol[1,2*e*]oxazoline-4*a*-carboxylate (DPX-MP062, Indoxycarb), from The Pesticide Manual, 11thEd. (1997), The British Crop Protection Council, London, page 453;
(CLXXXII) *N*'-tert.-butyl-*N*'-(3,5-dimethylbenzoyl)-3-methoxy-2-methylbenzohydrazide (RH-2485, Methoxyfenozide), from The Pesticide Manual, 11thEd. (1997), The British Crop Protection Council, London, page 1094; and
(CLXXXIII) (*N'*-[4-methoxy-biphenyl-3-yl]-hydrazinecarboxylic acid isopropylester (D 2341), from Brighton Crop Protection Conference, 1996, 487- 493;
(R2) Book of Abstracts, 212th ACS National Meeting Orlando, FL, August 25-29 (1996), AGRO-020. Publisher: American Chemical Society, Washington, D.C. CONEN: 63BFAF.

As a consequence of the above details, a further essential aspect of the present invention relates to combination preparations for the control of parasites on warm-blooded animals, characterised in that they contain, in addition to a compound of formula I, at least one further active ingredient having the same or different sphere of activity and at least one physiologically acceptable carrier. The present invention is not restricted to two-fold combinations.

As a rule, the insecticidal and acaricidal compositions according to the invention contain 0.1 to 99 % by weight, especially 0.1 to 95 % by weight of active ingredient of formula I, la or mixtures thereof, 99.9 to 1 % by weight, especially 99.8 to 5 % by weight of a solid or liquid admixture, including 0 to 25 % by weight, especially 0.1 to 25 % by weight of a surfactant.

Application of the compositions according to the invention to the animals to be treated may take place topically, perorally, parenterally or subcutaneously, the composition being present in the form of solutions, emulsions, suspensions, (drenches), powders, tablets, boli, capsules and pour-on formulations.

Preferred topical formulations are understood to refer to a ready-to-use solution in form of a spot-on, pour-on or spray-on formulation often consisting of a dispersion or suspoemulsion or a combination of active ingredient and spreading auxiliaries. The expression spot-on or pour-on method is understood to refer to a ready-to-use concentrate intended to be applied topically and locally on the animal. This sort of formulation is intended to be applied directly to a relatively small area of the sheep, preferably on the animal's back and breech or at several points along the line of the back and breech. It is applied as a low volume of about 0.05 to 1 ml per kg, preferably about 0.1 ml per kg, with a total volume from 10 to 100 ml per animal, preferably limited to a maximum of about 50 ml. However, it goes without saying that the total volume has to be adapted to the animal that is in need of the treatment and will clearly be different, for example, in young cats and in cattle. These pour-on and spot-on formulations are designed to spread all around the animal giving protection or treatment to almost any part of the animal. Even so the administration is carried out by applying a swab or spray of the pour-on or spot-on formulation to a relatively small area of the coat, one observes that from the active substance is dispersed almost automatically over wide areas of the fur owing to the spreading nature of the components in the formulation and assisted by the animal's movements.

Pour-on or spot-on formulations suitably contain carriers, which promote rapid dispersement over the skin surface or in the coat of the host animal, and are generally regarded as spreading oils. Suitable carriers are e.g. oily solutions; alcoholic and isopropanolic solutions such as solutions of 2-octyldodecanol or oleyl alcohol; solutions in esters of monocarboxylic acids, such as isopropyl myristate, isopropyl palmitate, lauric acid oxalate, oleic acid oleyl

ester, oleic acid decyl ester, hexyl laurate, oleyl oleate, decyl oleate, capric acid esters of saturated fat alcohols of chain length $C_{12}$-$C_{18}$; solutions of esters of dicarboxylic acids, such as dibutyl phthalate, diisopropyl isophthalate, adipic acid diisopropyl ester, di-n-butyl adipate or also solutions of esters of aliphatic acids, e.g. glycols. It may be advantageous for a dispersing agent to be additionally present, such as one known from the pharmaceutical or cosmetic industry. Examples are 2-pyrrolidone, 2-(N-alkyl)pyrrolidone, acetone, polyethylene glycol and the ethers and esters thereof, propylene glycol or synthetic triglycerides.

The oily solutions include e.g. vegetable oils such as olive oil, groundnut oil, sesame oil, pine oil, linseed oil or castor oil. The vegetable oils may also be present in epoxidised form. Paraffins and silicone oils may also be used.

A pour-on or spot-on formulation generally contains 1 to 20 % by weight of a compound of formula I, 0.1 to 50 % by weight of dispersing agent and 45 to 98.9 % by weight of solvent.

The pour-on or spot-on method is especially advantageous for use on herd animals such as cattle, horses, sheep or pigs, in which it is difficult or time-consuming to treat all the animals orally or by injection. Because of its simplicity, this method can of course also be used for all other animals, including individual domestic animals or pets, and is greatly favoured by the keepers of the animals, as it can often be carried out without the specialist presence of the veterinarian.

Whereas it is preferred to formulate commercial products as concentrates, the end user will often use dilute formulations. However, this depends on the mode of administration. Orally administered products are most often used in diluted form or as feed additives, whereas commercial pour-on and spot-on formulations are normally ready-to-use concentrates.

Such compositions may also contain further additives, such as stabilisers, anti-foaming agents, viscosity regulators, binding agents or tackifiers, as well as other active ingredients, in order to achieve special effects.

Insecticidal and acaricidal compositions of this type, which are used by the end user, similarly form a constituent of the present invention.

In each of the processes according to the invention for pest control or in each of the pest control compositions according to the invention, the active ingredients of formula I can be used in all of their steric configurations or in mixtures thereof.

The invention also includes a method of prophylactically protecting animals, especially productive livestock, domestic animals and pets, against parasitic helminths, which is characterised in that the active ingredients of formula I or the active ingredient formulations prepared therefrom are administered to the animals as an additive to the feed, or to the drinks or also in solid or liquid form, orally or by injection or parenterally. The invention also includes the compounds of formula I according to the invention for usage in one of the said processes.

The following examples serve merely to illustrate the invention without restricting it, the term active ingredient representing a substance listed in table 1.

In particular, preferred formulations are made up as follows:
(% = percent by weight)

| Formulation examples | | |
|---|---|---|
| 1. Granulate | a) | b) |
| active ingredient | 5 % | 10 % |
| kaolin | 94 % | - |
| highly dispersed silicic acid | 1 % | - |
| attapulgite | - | 90 % |

The active ingredient is dissolved in methylene chloride, sprayed onto the carrier and the solvent subsequently concentrated by evaporation under vacuum. Granulates of this kind can be mixed with the animal feed.

| 2. Granulate | |
|---|---|
| active ingredient | 3 % |
| polyethylene glycol (mw 200) | 3 % |
| kaolin | 94 % |
| (mw = molecular weight) | |

The finely ground active ingredient is evenly applied in a mixer to the kaolin which has been moistened with polyethylene glycol. In this way, dust-free coated granules are obtained.

| 3. Tablets or boli | | |
|---|---|---|
| I | active ingredient | 33.00 % |
| | methylcellulose | 0.80 % |
| | silicic acid, highly dispersed | 0.80 % |
| | corn starch | 8.40 % |
| II | lactose, cryst. | 22.50 % |
| | corn starch | 17.00 % |
| | microcryst. cellulose | 16.50 % |
| | magnesium stearate | 1.00 % |

I Methyl cellulose is stirred into water. After the material has swollen, silicic acid is stirred in and the mixture homogeneously suspended. The active ingredient and the corn starch are mixed. The aqueous suspension is worked into this mixture and kneaded to a dough. The resulting mass is granulated through a 12 M sieve and dried.

II All 4 excipients are mixed thoroughly.

III The preliminary mixes obtained according to I and II are mixed and pressed into tablets or boli.

| 4. Injectables | | |
|---|---|---|
| A. | Oily vehicle (slow release) | |
| 1. | active ingredient | 0.1-1.0 g |
| | groundnut oil | ad 100 ml |
| 2. | active ingredient | 0.1-1.0 g |
| | sesame oil | ad 100 ml |

Preparation: The active ingredient is dissolved in part of the oil whilst stirring and, if required, with gentle heating, then after cooling made up to the desired volume and sterile-filtered through a suitable membrane filter with a pore size of 0.22 μm.

| B Water-miscible solvent (average rate of release) | |
|---|---|
| active ingredient | 0.1-1.0 g |
| 4-hydroxymethyl-1,3-dioxolane (glycerol formal) | 40 g |
| 1,2-propanediol | ad 100 ml |
| active ingredient | 0.1-1.0 g |
| glycerol dimethyl ketal | 40 g |
| 1,2-propanediol | ad 100 ml |

Preparation: The active ingredient is dissolved in part of the solvent whilst stirring, made up to the desired volume and sterile-filtered through a suitable membrane filter with a pore size of 0.22 μm.

| C. Aqueous solubilisate (rapid release) | | |
|---|---|---|
| 1. | active ingredient | 0,1-1,0 g |
| | polyethoxylated castor oil (40 ethylene oxide units) | 10 g |
| | 1,2-propanediol | 20 g |
| | benzyl alcohol | 1 g |
| | aqua ad inject. | ad 100 ml |
| 2. | active ingredient | 0.1-1.0 g |
| | polyethoxylated sorbitan monooleate (20 ethylene oxide units) | 8 g |
| | 4-hydroxymethyl-1,3-dioxolane (glycerol formal) | 20 g |
| | benzyl alcohol | 1 g |

(continued)

| C. Aqueous solubilisate (rapid release) | | |
|---|---|---|
| | aqua ad inject. | ad 100 ml |

Preparation: The active ingredient is dissolved in the solvents and the surfactant, and made up with water to the desired volume. Sterile filtration through an appropriate membrane filter of 0.22 µm pore size.

| 5. Pour on | |
|---|---|
| A. | |
| active ingredient<br>isopropyl myristate<br>isopropanol | 5 g<br>10 g<br>ad 100 ml |
| B | |
| active ingredient<br>hexyl laurate<br>medium-chained triglyceride<br>ethanol | 2 g<br>5 g<br>15 g<br>ad 100 ml |
| C. | |
| active ingredient<br>oleyl oleate<br>N-methyl-pyrrolidone<br>isopropanol | 2 g<br>5 g<br>40 g<br>ad 100 ml |

The aqueous systems may also preferably be used for oral and/or intraruminal application.

The compositions may also contain further additives, such as stabilisers, e.g. where appropriate epoxidised vegetable oils (epoxidised coconut oil, rapeseed oil, or soybean oil); antifoams, e.g. silicone oil, preservatives, viscosity regulators, binders, tackifiers, as well as fertilisers or other active ingredients to achieve special effects.

Further biologically active substances or additives, which are neutral towards the compounds of formula I and do not have a harmful effect on the host animal to be treated, as well as mineral salts or vitamins, may also be added to the described compositions.

The following examples serve to illustrate the invention. They do not limit the invention. The letter 'h' stands for hour. The starting substances used may be produced by methods described in literature or are commercially available.

Preparation examples

Example 1: 4,6-bis-(4-fluoro-3-methylphenoxy)-pyrimidin-5-ylamine

In 1 ml DMF 154 mg 4-fluoro-3-methyl-phenol are dissolved and 30 mg sodium hydride is added slowly. The reaction mixture is stirred for 1 h at room temperature and then 0.5ml of a DMF stock solution containing 66 mg 4,6-Dichloro-5-aminopyrimidine is added in one portion. The resulting reaction mixture is stirred and heated for 2 h at 100°C and an additional for 18 h at 80°C. The reaction mixture is allowed to cool to room temperature. The precipitate is filtered using PE filter cartouches and washed with 2 ml acetonitrile. The crude mixture is finally purified using preparative reversed phase chromatography on a Daisogel C18-ODS AP column with a water/formic acid (10'000:1) to acetonitrile/ formic acid (10'000:1) gradient. The title compound is isolated by removal of the solvent.

Example 2: 4,6-bis-(3-fluorophenoxy)-pyrimidin-5-ylamine

A mixture of 171 mg 3-fluorophenol and 1.38 g potassium carbonate is stirred together, then 1ml of DMF stock solution containing 84 mg 4,6-Dichloro-5-aminopyrimidine are added, followed by an additional 1 ml DMF. The resulting reaction mixture is stirred for 18 h at 80°C. The reaction mixture is then allowed to cool to room temperature. The precipitate is filtered using PE filter cartouches and washed with 2 ml acetonitrile. The crude residue is purified by

preparative reversed phase chromatography on a Daisogel C18-ODS AP column with a water/formic acid (10'000:1) to acetonitrile/ formic acid (10'000:1) gradient. The title compound is isolated by removal of the solvent.

Example 3: 4,6-bis-(4-fluoro-3-(trifluoromethyl)phenoxy)-pyrimidin-5-ylamine

Dissolved in 70 ml DMF, 40.8 g 4-fluoro-3-(trifluoromethyl)-phenol are stirred under an inert gas atmosphere and cooled to 10°C. To this, 5.8 g sodium hydride is added slowly under vigorous stirring. The mixture is then allowed to cool to room temperature and stirred for 1 h. Then, a solution of 19.9 g 4,6-Dichloro-5-aminopyrimidine in 50 ml DMF is added dropwise and the reaction mixture is heated for 24 h at 80°C. After quenching with water and concentration under reduced pressure the crude mixture is extracted twice with ethyl acetate. The combined organic phases are washed with water and saturated sodium chloride and finally dried over magnesium sulfate and charcoal. The dark brown oily residue is dissolved in 100 ml diethylether and treated with 100 ml hexane. The resulting title compound crystallizes as a colorless solid with a melting point of 105-106°C.

Example 4: [4,6-bis-(4-fluoro-3-(trifluoromethyl)phenoxy)]-pyrimidin-5-yl-acetamide

In 2 ml dichloromethane and 57 mg Ethyldiisopropylamine and 100 mg 4,6-bis-(4-fluoro-3-(trifluoromethyl) phenoxy)]-5-aminopyrimidine are dissolved and treated with 3.2 mg dimethylaminopyridine and 41 mg acetic acid anhydride. The reaction mixture is stirred at room temperature for 72 h, the solvent evaporated, the residue recovered in ethyl acetate and extracted with 2 ml 1 N hydrochloric acid, saturated sodium bicarbonate and brine and finally dried over magnesium sulfate. The organic layer is evaporated and the resulting solid purified by column chromatography (eluents: dichloromethane, ethyl acetate). Removal of the solvent yields the title compound.

Example 5: 4,6-[bis-(4-fluoro-3-(trifluoromethyl)phenoxy)]-pyrimidin-5-yl-carbamic acid ethyl ester

In 2 ml pyridine 100 mg 4,6-bis-(4-fluoro-3-(trifluoromethyl)phenoxy)]-5-aminopyrimidine are dissolved and treated with 44 mg ethyl chloroformate. The reaction mixture is stirred at room temperature for 18 h. Then, 2 ml 2N hydrochloric acid are added and the mixture extracted three times with diethylether. The combined organic layers are washed with saturated sodium bicarbonate and brine and finally dried over magnesium sulfate. After removal of the solvent the crude residue is purified by preparative normal-phase HPLC, yielding the title compound.

Example 6: 4,6-[bis-(3-(trifluoromethyl)phenylamino)]-pyrimidin-5-ylamine

In 10 ml tetrahydrofurane/water (1:1) 1.0 g of 3-(trifluoromethyl)aniline and 0.5 g of 5-nitro-4,6-dichloropyrimidine are added, followed by 2 drops of concentrated hydrochloric acid. The reaction mixture is stirred under reflux for 18h, cooled to room temperature, then 2.5 g of tin dichloride are added. The mixture is stirred under reflux for 18h, evaporated under reduced pressure and ethylacetate is added. The organic phase is washed with saturated sodium bicarbonate and brine, and finally dried over magnesium sulphate. After removal of the solvent the residue is purified by preparative reverse phase chromatography on a Daisogel C18-ODS AP column with a water/formic acid (10'000:1) to acetonitrile/ formic acid (10'000:1) gradient. The title compound is isolated by removal of the solvent as a solid.

The substances named in the following table may also be prepared analogously to the above-described method.

Table 1

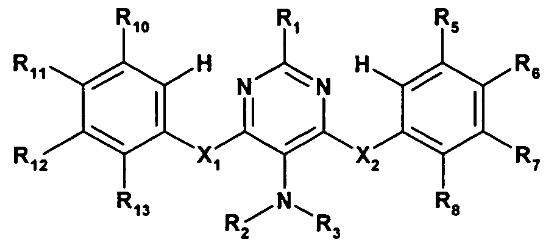

| No | $X_{1/2}$ | $R_1$ | $R_2$ | $R_3$ | R5 / R10 | R6 / R11 | R7 / R12 | R8 / R13 | mp (°C) |
|---|---|---|---|---|---|---|---|---|---|
| 1.1 | O | H | H | H | H | F | Cl | H | 134-135 |
| 1.2 | O | H | H | H | $CF_3$ | H | H | F | 112-115 |
| 1.3 | O | H | H | H | H | F | F | H | 128-129 |
| 1.4 | O | H | H | H | H | H | F | H | 110-112 |
| 1.5 | O | H | H | H | H | H | $CF_3$ | H | 81-83 |
| 1.6 | O | H | H | H | H | Cl | H | H | 140-141 |
| 1.7 | O | H | H | H | F | F | H | F | 128-129 |
| 1.8 | O | H | H | H | H | H | H | Cl | 179-182 |
| 1.9 | O | H | H | H | H | F | $CF_3$ | H | 100-106 |
| 1.10 | O | H | H | H | H | F | H | H | 164-166 |
| 1.11 | O | H | H | H | H | Cl | Cl | H | 177-179 |
| 1.12 | O | H | H | H | H | Cl | F | H | 150-153 |
| 1.13 | O | H | H | H | H | $NO_2$ | H | H | |
| 1.14 | O | H | H | H | H | H | Cl | H | 115-116 |
| 1.15 | O | H | H | H | H | $CF_3$ | H | H | 130-132 |
| 1.16 | O | $OCH_2CH_3$ | H | H | H | F | $CF_3$ | H | 96-98 |
| 1.17 | O | H | H | H | H | H | H | H | 145-146 |
| 1.18 | O | H | H | H | H | $CH_3$ | H | H | 170-171 |
| 1.19 | O | H | H | H | H | CN | H | Cl | |
| 1.20 | O | H | H | H | H | F | $CH_3$ | H | 155-156 |
| 1.21 | O | H | H | H | H | Cl | $NO_2$ | H | 198-200 |
| 1.22 | O | H | H | H | H | H | $OCF_3$ | H | Oil |
| 1.23 | O | H | H | H | H | H | Br | H | 121-123 |
| 1.24 | O | H | H | H | H | H | $OCH_3$ | H | 148-149 |
| 1.25 | S | H | H | H | H | $CF_3$ | H | H | 194-196 |
| 1.26 | O | H | H | H | H | CN | H | H | 241-242 |
| 1.27 | O | H | H | H | H | $OCH_{33}$ | H | H | 189-191 |
| 1.28 | O | H | H | $C(O)OCH_2CH_3$ | H | F | $CF_3$ | H | 114-115 |
| 1.29 | O | H | H | $COCH_3$ | H | F | $CF_3$ | H | 122-124 |
| 1.30 | O | H | $C(O)O\ CH_2CH_3$ | $C(O)O\ CH_2CH_3$ | H | F | $CF_3$ | H | 167-169 |
| 1.31 | NH | H | H | H | H | F | $CF_3$ | H | 199-201 |
| 1.32 | NH | H | H | CHO | H | F | $CF_3$ | H | 223-228 |
| 1.33 | NH | H | H | H | H | Cl | Cl | H | 222-226 |

| | | | | | | | | | |
|---|---|---|---|---|---|---|---|---|---|
| 1.34 | NH | H | H | H | H | $CF_3$ | H | H | 207-212 |
| 1.35 | NH | H | H | H | H | F | H | $CF_3$ | 156-159 |
| 1.36 | O | H | H | CHO | H | F | $CF_3$ | H | 182-186 |
| 1.37 | NH | H | H | H | H | H | $CF_3$ | H | 171-175 |
| 1.38 | O | H | H | CHO | H | F | Cl | H | 128-133 |
| 1.39 | O | H | H | H | H | Cl | $CF_3$ | H | Oil |
| 1.40 | NH | H | H | H | H | Cl | $CF_3$ | H | 230-234 |
| 1.41 | NH | H | H | H | H | H | $OCF_3$ | H | 133-140 |
| 1.42 | O | H | H | Bn | H | F | $CF_3$ | H | |
| 1.43 | O | H | H | C(O)N $CH_2CH_3$ | H | F | $CF_3$ | H | |
| 1.44 | O | H | H | $CH_3$ | H | F | $CF_3$ | H | |
| 1.45 | O | H | H | H | H | H | $NO_2$ | H | 206-208 |
| 1.46 | O | H | $CH_3$ | $CH_3$ | H | F | $CF_3$ | H | |
| 1.47 | O | H | H | H | H | H | H | $OCH_3$ | 187-188 |
| 1.48 | O | H | H | H | H | -$OCH_2O$- | | H | 179-180 |
| 1.49 | O | H | O | O | H | F | $CF_3$ | H | |
| 1.50 | O | H | Bn | Bn | H | F | $CF_3$ | H | |

Table 2

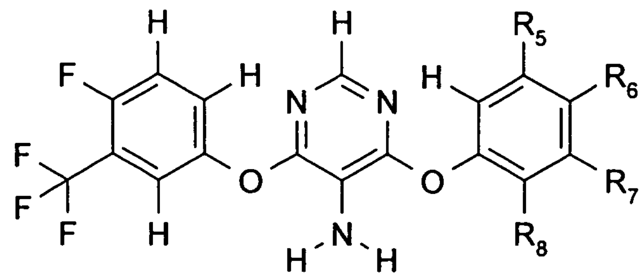

| No | $R_5$ | $R_6$ | $R_7$ | $R_8$ | mp (°C) |
|---|---|---|---|---|---|
| 2.1 | Cl | F | H | H | 132-135 |
| 2.2 | F | F | H | H | 101-103 |
| 2.3 | H | $CH_3$ | H | $CH_3$ | 101-103 |
| 2.4 | H | F | H | H | 85-87 |
| 2.5 | H | F | H | H | 97-98 |
| 2.6 | H | F | $CH_3$ | H | 109-110 |
| 2.7 | H | H | H | H | 83-85 |
| 2.8 | $CF_3$ | H | H | F | Oil |
| 2.9 | $CF_3$ | H | H | H | Oil |
| 2.10 | H | Cl | $CF_3$ | H | Oil |
| 2.11 | H | H | H | $CF_3$ | Oil |
| 2.12 | H | $CF_3$ | H | H | 107-109 |
| 2.13 | F | H | H | H | 91-92 |
| 2.14 | CH3 | Cl | H | H | 111-112 |
| 2.15 | H | $OCH_3$ | H | H | 126-127 |
| 2.16 | H | H | H | $OCF_3$ | Oil |
| 2.17 | Cl | Cl | H | H | 102-103 |
| 2.18 | H | $CH_3$ | H | H | 118-119 |
| 2.19 | H | H | H | $OCH_3$ | 85-86 |

Biological Examples:

1. Activity *in vitro* against *Dermanyssus gallinae* (Chicken red mite).

Purified female mite population is used to seed an High Throughput Screening (HTS) format plate (96 well plate) containing substances to be evaluated for anti-parasitic activity. Each compound is evaluated in a serial dilution manner in order to determine the Minimal Efficacious Dose (MED). Mites are left in contact with the compound for 10 minutes. Mites are then incubated at 25°C, 60% relative humidity (RH) for 5 days, where the activity is recorded. Acaricidal activity is confirmed if mites are dead without having laid eggs. Egg laying and further development are also recorded in order to identifying growth-regulating activity.

2. Activity *in vitro* against *Ctenocephalides felis* (Cat flea).

Mixed adult population of fleas are put into an High Throughput Screening (HTS) plate format (96 well plate) allowing fleas to get access to treated blood via an artificial feeding system. Each compound is evaluated in a serial dilution manner in order to determine the Minimal Efficacious Dose (MED). Fleas are fed on treated blood for 24 hours,

where the anti-parasitic activity is recorded. Insecticidal activity is observed when dead fleas are recovered from the feeding system.

The compounds number 1.2, 1.3, 1.7, 1.9, 1.22, 1.36, 2.1 2.2, 2.4, 2.11, 2.13, 2.14 and 2.17 show in the HTS insecticidal or acaricidal efficacy of more than 80%. Especially 1.7, 1.9, 1.22 showed efficacy against *Ctenocephalides felis* of more than 80% at 100ppm.

## Claims

**1.** Use of a compound of formula

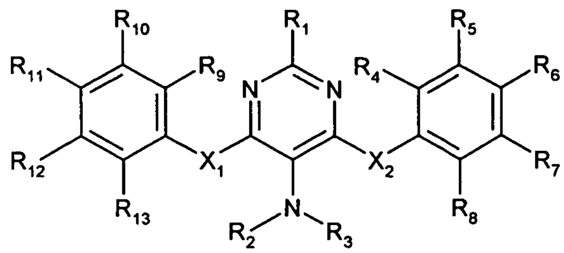

I,

wherein

$R_1$ is hydrogen, halogen, cyano, OH, SH, $NO_2$, COOH, $COOR_2$, $CONH_2$, $CONR_2R_3$, $SO_3H$, $SO_2NR_2R_3$, $C_1$-$C_6$-alkyl, halo-$C_1$-$C_6$-alkyl, $C_1$-$C_6$-alkoxy, halo-$C_1$-$C_6$-alkoxy, $C_2$-$C_6$-alkenyl, halo-$C_2$-$C_6$-alkenyl, $C_2$-$C_6$-alkinyl, $C_3$-$C_6$-cycloalkyl, halo-$C_3$-$C_6$-cycloalkyl, $C_3$-$C_6$-cyclo-alkyloxy, $C_3$-$C_6$-cycloalkylthio, $C_2$-$C_6$-alkenyloxy, halo-$C_2$-$C_6$-alkenyloxy, $C_1$-$C_6$-alkylthio, halo-$C_1$-$C_6$-alkylthio, $C_1$-$C_6$-alkylsulfonyloxy, halo-$C_1$-$C_6$-alkylsulfonyloxy, $C_1$-$C_6$-alkylsulfinyl, halo-$C_1$-$C_6$-alkylsulfinyl, $C_1$-$C_6$-alkylsulfonyl, halo-$C_1$-$C_6$-alkylsulfonyl, $C_2$-$C_6$-alkenylthio, halo-$C_2$-$C_6$-alkenylthio, $C_2$-$C_6$-alkenylsulfinyl, halo-$C_2$-$C_6$-alkenylsulfinyl, $C_2$-$C_6$-alkenylsulfonyl, halo-$C_2$-$C_6$-alkenylsulfonyl, $NR_2R_3$, unsubstituted or one- to five-fold substituted aryl or unsubstituted or substituted hetaryl, the substituents selected from the group consisting of halogen, cyano, OH, SH, $NO_2$, COOH, $COOR_2$, $CONH_2$, $CONR_2R_3$, $SO_3H$, $SO_2NR_2R_3$, $C_1$-$C_6$-alkyl, halo-$C_1$-$C_6$-alkyl, $C_1$-$C_6$-alkoxy, halo-$C_1$-$C_6$-alkoxy, $C_2$-$C_6$-alkenyl, halo-$C_2$-$C_6$-alkenyl, $C_2$-$C_6$-alkinyl, $C_3$-$C_6$-cycloalkyl, halo-$C_3$-$C_6$-cycloalkyl, $C_3$-$C_6$-cycloalkyloxy, $C_3$-$C_6$-cycloalkylthio, $C_2$-$C_6$-alkenyloxy, halo-$C_2$-$C_6$-alkenyloxy, $C_1$-$C_6$-alkylthio, halo-$C_1$-$C_6$-alkylthio, $C_1$-$C_6$-alkylsulfonyloxy, halo-$C_1$-$C_6$-alkylsulfonyloxy, $C_1$-$C_6$-alkylsulfinyl, halo-$C_1$-$C_6$-alkylsulfinyl, $C_1$-$C_6$-alkylsulfonyl, halo-$C_1$-$C_6$-alkylsulfonyl, $C_2$-$C_6$-alkenylthio, halo-$C_2$-$C_6$-alkenylthio, $C_2$-$C_6$-alkenylsulfinyl, halo-$C_2$-$C_6$-alkenylsulfinyl, $C_2$-$C_6$-alkenylsulfonyl, halo-$C_2$-$C_6$-alkenylsulfonyl and $NR_2R_3$;

$R_2$ and $R_3$, independently of one another, signify hydrogen, $C_1$-$C_6$-alkyl, halo-$C_1$-$C_6$-alkyl, formyl, $C_1$-$C_6$-alkylcarbonyl, halo-$C_1$-$C_6$-alkylcarbonyl, $C_1$-$C_6$-alkoxycarbonyl, halo-$C_1$-$C_6$-alkoxycarbonyl, $C_1$-$C_6$-alkylaminocarbonyl, di-$C_1$-$C_6$-alkylaminocarbonyl or unsubstituted or one- to five-fold substituted benzyl, the substituents selected from the group consisting of halogen, cyano, OH, SH, $NO_2$, COOH, $COOR_2$, $CONH_2$, $CONR_2R_3$, $SO_3H$, $SO_2NR_2R_3$, $C_1$-$C_6$-alkyl, halo-$C_1$-$C_6$-alkyl, $C_1$-$C_6$-alkoxy, halo-$C_1$-$C_6$-alkoxy, $C_2$-$C_6$-alkenyl, halo-$C_2$-$C_6$-alkenyl, $C_2$-$C_6$-alkinyl, $C_3$-$C_6$-cycloalkyl, halo-$C_3$-$C_6$-cycloalkyl, $C_3$-$C_6$-cycloalkyloxy, $C_3$-$C_6$-cycloalkylthio, $C_2$-$C_6$-alkenyloxy, halo-$C_2$-$C_6$-alkenyloxy, $C_1$-$C_6$-alkylthio, halo-$C_1$-$C_6$-alkylthio, $C_1$-$C_6$-alkylsulfonyloxy, halo-$C_1$-$C_6$-alkylsulfonyloxy, $C_1$-$C_6$-alkylsulfinyl, halo-$C_1$-$C_6$-alkylsulfinyl, $C_1$-$C_6$-alkylsulfonyl, halo-$C_1$-$C_6$-alkylsulfonyl, $C_2$-$C_6$-alkenylthio, halo-$C_2$-$C_6$-alkenylthio, $C_2$-$C_6$-alkenylsulfinyl, halo-$C_2$-$C_6$-alkenylsulfinyl, $C_2$-$C_6$-alkenylsulfonyl and halo-$C_2$-$C_6$-alkenylsulfonyl;

$R_4$, $R_5$, $R_6$, $R_7$, $R_8$, $R_9$, $R_{10}$, $R_{11}$, $R_{12}$ and $R_{13}$, independently of one another, are hydrogen, halogen, cyano, nitro, OH, SH, $NO_2$, COOH, $COOR_2$, $CONH_2$, $CONR_2R_3$, $SO_3H$, $SO_2NR_2R_3$, $C_1$-$C_6$-alkyl, halo-$C_1$-$C_6$-alkyl, $C_1$-$C_6$-alkoxy, halo-$C_1$-$C_6$-alkoxy, $C_2$-$C_6$-alkenyl, halo-$C_2$-$C_6$-alkenyl, $C_2$-$C_6$-alkinyl, $C_3$-$C_6$-cycloalkyl, $C_2$-$C_6$-alkenyloxy, halo-$C_2$-$C_6$-alkenyloxy, $C_1$-$C_6$-alkylthio, halo-$C_1$-$C_6$-alkylthio, $C_1$-$C_6$-alkylsulfonyloxy, halo-$C_1$-$C_6$-alkylsulfonyloxy, $C_1$-$C_6$-alkylsulfinyl, halo-$C_1$-$C_6$-alkylsulfinyl, $C_1$-$C_6$-alkylsulfonyl, halo-$C_1$-$C_6$-alkylsulfonyl, $C_2$-$C_6$-alkenylthio, halo-$C_2$-$C_6$-alkenylthio, $C_2$-$C_6$-alkenylsulfinyl, halo-$C_2$-$C_6$-alkenylsulfinyl, $C_2$-$C_6$-alkenylsulfonyl, halo-$C_2$-$C_6$-alkenylsulfonyl, $C_1$-$C_6$-alkylamino, di-$C_1$-$C_6$-alkylamino, $C_1$-$C_6$-alkylsulfonylamino, halo-$C_1$-$C_6$-alkylsulfonylamino, $C_1$-$C_6$-alkylcarbonyl, halo-$C_1$-$C_6$-alkylcarbonyl, $C_1$-$C_6$-alkoxycarbonyl, $C_1$-$C_6$-alkylaminocarbonyl, di-$C_1$-$C_6$-alkylaminocarbonyl, or unsubstituted or one- to five-fold substituted aryl or unsubstituted or substituted hetaryl, the substituents selected from the group consisting of halogen, cyano, OH, SH, $NO_2$, COOH, $COOR_2$, $CONH_2$, $CONR_2R_3$, $SO_3H$, $SO_2NR_2R_3$, $C_1$-$C_6$-alkyl, halo-$C_1$-$C_6$-alkyl, $C_1$-$C_6$-alkoxy, halo-$C_1$-$C_6$-alkoxy, $C_2$-$C_6$-alkenyl, halO-$C_2$-$C_6$-alkenyl, $C_2$-$C_6$-alkinyl, $C_3$-$C_6$-cycloalkyl, halo-$C_3$-$C_6$-cycloalkyl, $C_3$-$C_6$-cycloalkyloxy, $C_3$-$C_6$-cycloalkylthio, $C_2$-$C_6$-alkenyloxy, halo-$C_2$-$C_6$-alkenyloxy, $C_1$-$C_6$-alkylthio, halo-$C_1$-$C_6$-alkylthio, $C_1$-$C_6$-alkylsulfonyloxy, halo-$C_1$-$C_6$-alkylsulfonyloxy, $C_1$-$C_6$-alkylsulfinyl, halo-$C_1$-$C_6$-alkylsulfinyl, $C_1$-$C_6$-alkylsulfonyl, halo-$C_1$-$C_6$-alkylsulfonyl, $C_2$-$C_6$-alkenylthio, halo-$C_2$-$C_6$-alkenylthio, $C_2$-$C_6$-alkenylsulfinyl, halo-$C_2$-$C_6$-alkenylsulfinyl, $C_2$-$C_6$-alkenylsulfonyl, halo-$C_2$-$C_6$-alkenylsulfonyl and $NR_2R_3$;

$X_1$ and $X_2$, independently of one another, are $C(R_{14})(R_{15})$, $N(R_{14})(R_{15})$, O, S, SO or $SO_2$ and

$R_{14}$ and $R_{15}$, independently of one another, signify hydrogen, $C_1$-$C_6$-alkyl, formyl, $C_1$-$C_6$-alkylcarbonyl or halo-$C_1$-$C_6$-alkylcarbonyl;

in the control of ectoparasites on animals.

**2.** Use of a compound of formula according to claim 1, wherein
$R_1$ is hydrogen, halogen, $C_1$-$C_6$-alkyl, halo-$C_1$-$C_6$-alkyl, $C_1$-$C_6$-alkoxy, halo-$C_1$-$C_6$-alkoxy, $C_3$-$C_6$-cycloalkyl, halo-$C_3$-$C_6$-cycloalkyl, $C_3$-$C_6$-cycloalkyloxy, $C_3$-$C_6$-cycloalkylthio, $C_1$-$C_6$-alkylthio or halo-$C_1$-$C_6$-alkylthio.

**3.** Use of a compound of formula according to claim 1, wherein
$R_1$ is hydrogen, halogen, $C_1$-$C_6$-alkyl, halo-$C_1$-$C_6$-alkyl, $C_1$-$C_6$-alkoxy or halo-$C_1$-$C_6$-alkoxy.

**4.** Use of a compound of formula according to claim 1, wherein
$R_1$ is hydrogen, $C_1$-$C_6$-alkyl or $C_1$-$C_6$-alkoxy.

**5.** Use of a compound of formula according to claim 1, wherein
$R_2$ and $R_3$, independently of one another, signify hydrogen, $C_1$-$C_6$-alkyl, formyl, $C_1$-$C_6$-alkylcarbonyl, $C_1$-$C_6$-alkoxycarbonyl, $C_1$-$C_6$-alkylaminocarbonyl, di-$C_1$-$C_6$-alkylaminocarbonyl or unsubstituted or one- to five-fold substituted benzyl, the substituents selected from the group consisting of halogen, cyano, OH, SH, $NO_2$, COOH, $COOR_2$, $CONH_2$, $CONR_2R_3$, $SO_3H$, $SO_2NR_2R_3$, $C_1$-$C_6$-alkyl, halo-$C_1$-$C_6$-alkyl, $C_1$-$C_6$-alkoxy, halo-$C_1$-$C_6$-alkoxy, $C_2$-$C_6$-alkenyl, halo-$C_2$-$C_6$-alkenyl, $C_2$-$C_6$-alkinyl, $C_3$-$C_6$-cycloalkyl, halo-$C_3$-$C_6$-cycloalkyl, $C_3$-$C_6$-cycloalkyloxy, $C_3$-$C_6$-cycloalkylthio, $C_2$-$C_6$-alkenyloxy, halo-$C_2$-$C_6$-alkenyloxy, $C_1$-$C_6$-alkylthio, halo-$C_1$-$C_6$-alkylthio, $C_1$-$C_6$-alkylsulfonyloxy, halo-$C_1$-$C_6$-alkylsulfonyloxy, $C_1$-$C_6$-alkylsulfinyl, halo-$C_1$-$C_6$-alkylsulfinyl, $C_1$-$C_6$-alkylsulfonyl, halo-$C_1$-$C_6$-alkylsulfonyl, $C_2$-$C_6$-alkenylthio, halo-$C_2$-$C_6$-alkenylthio, $C_2$-$C_6$-alkenylsulfinyl, halo-$C_2$-$C_6$-alkenylsulfinyl, $C_2$-$C_6$-alkenylsulfonyl and halo-$C_2$-$C_6$-alkenylsulfonyl.

**6.** Use of a compound of formula according to claim 1, wherein
$R_2$ and $R_3$, independently of one another, signify hydrogen, $C_1$-$C_4$-alkyl, formyl, $C_1$-$C_4$-alkylcarbonyl or benzyl.

**7.** Use of a compound of formula according to claim 1, wherein
$R_2$ and $R_3$, independently of one another, signify hydrogen, $C_1$-$C_2$-alkyl or formyl.

**8.** Use of a compound of formula according to claim 1, wherein
$R_4$, $R_5$, $R_6$, $R_7$, $R_8$, $R_9$, $R_{10}$, $R_{11}$, $R_{12}$ and $R_{13}$, independently of one another, are hydrogen, halogen, cyano, nitro, $C_1$-$C_6$-alkyl, halo-$C_1$-$C_6$-alkyl, $C_1$-$C_6$-alkoxy, halo-$C_1$-$C_6$-alkoxy, $C_3$-$C_6$-cycloalkyl, $C_1$-$C_6$-alkylthio, halo-$C_1$-$C_6$-alkylthio or unsubstituted or one- to five-fold substituted aryl or unsubstituted or substituted hetaryl, the substituents selected from the group consisting of halogen, cyano, OH, SH, $NO_2$, COOH, $COOR_2$, $CONH_2$, $CONR_2R_3$, $SO_3H$, $SO_2NR_2R_3$, $C_1$-$C_6$-alkyl, halo-$C_1$-$C_6$-alkyl, $C_1$-$C_6$-alkoxy, halo-$C_1$-$C_6$-alkoxy, $C_2$-$C_6$-alkenyl, halo-$C_2$-$C_6$-alkenyl, $C_2$-$C_6$-alkinyl, $C_3$-$C_6$-cycloalkyl, halo-$C_3$-$C_6$-cycloalkyl, $C_3$-$C_6$-cycloalkyloxy, $C_3$-$C_6$-cycloalkylthio, $C_2$-$C_6$-alkenyloxy, halo-$C_2$-$C_6$-alkenyloxy, $C_1$-$C_6$-alkylthio, halo-$C_1$-$C_6$-alkylthio, $C_1$-$C_6$-alkylsulfonyloxy, halo-$C_1$-$C_6$-alkylsulfonyloxy, $C_1$-$C_6$-alkylsulfinyl, halo-$C_1$-$C_6$-alkylsulfinyl, $C_1$-$C_6$-alkylsulfonyl, halo-$C_1$-$C_6$-alkylsulfonyl, $C_2$-$C_6$-alkenylthio, halo-$C_2$-$C_6$-alkenylthio, $C_2$-$C_6$-alkenylsulfinyl, halo-$C_2$-$C_6$-alkenylsulfinyl, $C_2$-$C_6$-alkenylsulfonyl, halo-$C_2$-$C_6$-alkenylsulfonyl and $NR_2R_3$;

**9.** Use of a compound of formula according to claim 1, wherein
$R_4$, $R_5$, $R_6$, $R_7$, $R_8$, $R_9$, $R_{10}$, $R_{11}$, $R_{12}$ and $R_{13}$, independently of one another, are hydrogen, halogen, $C_1$-$C_4$-alkyl, halo-$C_1$-$C_4$-alkyl, $C_1$-$C_4$-alkoxy or halo-$C_1$-$C_4$-alkoxy.

**10.** Use of a compound of formula according to claim 1, wherein
$R_4$, $R_5$, $R_6$, $R_7$, $R_8$, $R_9$, $R_{10}$, $R_{11}$, $R_{12}$ and $R_{13}$, independently of one another, are hydrogen, halogen, $C_1$-$C_2$-alkyl or halo-$C_1$-$C_2$-alkyl.

**11.** Use of a compound of formula according to claim 1, wherein
$R_4$, $R_5$, $R_6$, $R_7$, $R_8$, $R_9$, $R_{10}$, $R_{11}$, $R_{12}$ and $R_{13}$, independently of one another, are hydrogen, halogen or $CF_3$.

**12.** Use of a compound of formula according to claim 1, wherein
$X_1$ and $X_2$, independently of one another, are $N(R_{14})(R_{15})$, O or S.

**13.** Use of a compound of formula according to claim 1, wherein
$X_1$ and $X_2$, independently of one another, are $NH_2$, O or S.

**14.** Use of a compound of formula according to claim 1, wherein
$X_1$ and $X_2$ are O.

**15.** Use of a compound of formula according to claim 1, wherein
$R_{14}$ and $R_{15}$, independently of one another, signify hydrogen, $C_1$-$C_4$-alkyl, formyl, $C_1$-$C_4$-alkylcarbonyl.

**16.** Use of a compound of formula according to claim 1, wherein
$R_{14}$ and $R_{15}$, independently of one another, signify hydrogen or $C_1$-$C_4$-alkyl.

**17.** Use of a compound of formula according to claim 1, wherein
$R_{14}$ and $R_{15}$ signify hydrogen.

**18.** Use of a compound of formula according to claim 1, wherein
$R_1$ is hydrogen, halogen, $C_1$-$C_6$-alkyl, halo-$C_1$-$C_6$-alkyl, $C_1$-$C_6$-alkoxy, halo-$C_1$-$C_6$-alkoxy, $C_3$-$C_6$-cycloalkyl, halo-$C_3$-$C_6$-cycloalkyl, $C_3$-$C_6$-cycloalkyloxy, $C_3$-$C_6$-cycloalkylthio, $C_1$-$C_6$-alkylthio or halo-$C_1$-$C_6$-alkylthio;
$R_2$ and $R_3$, independently of one another, signify hydrogen, $C_1$-$C_6$-alkyl, formyl, $C_1$-$C_6$-alkylcarbonyl, $C_1$-$C_6$-alkoxycarbonyl, $C_1$-$C_6$-alkylaminocarbonyl, di-$C_1$-$C_6$-alkylaminocarbonyl or benzyl;
$R_4$, $R_5$, $R_6$, $R_7$, $R_8$, $R_9$, $R_{10}$, $R_{11}$, $R_{12}$ and $R_{13}$, independently of one another, are hydrogen, halogen, cyano, nitro, $C_1$-$C_6$-alkyl, halo-$C_1$-$C_6$-alkyl, $C_1$-$C_6$-alkoxy, halo-$C_1$-$C_6$-alkoxy, $C_3$-$C_6$-cycloalkyl, $C_1$-$C_6$-alkylthio, halo-$C_1$-$C_6$-alkylthio or unsubstituted or one- to five-fold substituted aryl or unsubstituted or substituted hetaryl, the substituents selected from the group consisting of halogen, cyano, OH, SH, $NO_2$, COOH, $COOR_2$, $CONH_2$, $CONR_2R_3$, $SO_3H$, $SO_2NR_2R_3$, $C_1$-$C_6$-alkyl, halo-$C_1$-$C_6$-alkyl, $C_1$-$C_6$-alkoxy, halo-$C_1$-$C_6$-alkoxy, $C_2$-$C_6$-alkenyl, halo-$C_2$-$C_6$-alkenyl, $C_2$-$C_6$-alkinyl, $C_3$-$C_6$-cycloalkyl, halo-$C_3$-$C_6$-cycloalkyl, $C_3$-$C_6$-cycloalkyloxy, $C_3$-$C_6$-cycloalkylthio, $C_2$-$C_6$-alkenyloxy, halo-$C_2$-$C_6$-alkenyloxy, $C_1$-$C_6$-alkylthio, halo-$C_1$-$C_6$-alkylthio, $C_1$-$C_6$-alkylsulfonyloxy, halo-$C_1$-$C_6$-alkylsulfonyloxy, $C_1$-$C_6$-alkylsulfinyl, halo-$C_1$-$C_6$-alkylsulfinyl, $C_1$-$C_6$-alkylsulfonyl, halo-$C_1$-$C_6$-alkylsulfonyl, $C_2$-$C_6$-alkenylthio, halo-$C_2$-$C_6$-alkenylthio, $C_2$-$C_6$-alkenylsulfinyl, halo-$C_2$-$C_6$-alkenylsulfinyl, $C_2$-$C_6$-alkenylsulfonyl, halo-$C_2$-$C_6$-alkenylsulfonyl and $NR_2R_3$;
$X_1$ and $X_2$, independently of one another, are $N(R_{14})(R_{15})$, O or S; and
$R_{14}$ and $R_{15}$, independently of one another, signify hydrogen, $C_1$-$C_4$-alkyl, formyl, $C_1$-$C_4$-alkylcarbonyl.

**19.** Use of a compound of formula according to claim 1, wherein
$R_1$ is hydrogen, halogen, $C_1$-$C_6$-alkyl, halo-$C_1$-$C_6$-alkyl, $C_1$-$C_6$-alkoxy or halo-$C_1$-$C_6$-alkoxy;
$R_2$ and $R_3$, independently of one another, signify hydrogen, $C_1$-$C_4$-alkyl, formyl, $C_1$-$C_4$-alkylcarbonyl or benzyl;
$R_4$, $R_5$, $R_6$, $R_7$, $R_8$, $R_9$, $R_{10}$, $R_{11}$, $R_{12}$ and $R_{13}$, independently of one another, are hydrogen, halogen, $C_1$-$C_4$-alkyl, halo-$C_1$-$C_4$-alkyl, $C_1$-$C_4$-alkoxy or halo-$C_1$-$C_4$-alkoxy; and
$X_1$ and $X_2$, independently of one another, are $NH_2$, O or S.

**20.** Use of a compound of formula according to claim 1, wherein
$R_1$ is hydrogen, $C_1$-$C_6$-alkyl or $C_1$-$C_6$-alkoxy;
$R_2$ and $R_3$, independently of one another, signify hydrogen, $C_1$-$C_2$-alkyl or formyl;
$R_4$, $R_5$, $R_6$, $R_7$, $R_8$, $R_9$, $R_{10}$, $R_{11}$, $R_{12}$ and $R_{13}$, independently of one another, are hydrogen, halogen, $C_1$-$C_2$-alkyl or halo-$C_1$-$C_2$-alkyl; and
$X_1$ and $X_2$ are O.

**21.** Use of a compound of formula according to claim 1, wherein

$R_1$ is hydrogen, $C_1$-$C_6$-alkyl or $C_1$-$C_6$-alkoxy;
$R_2$ and $R_3$, independently of one another, signify hydrogen, $C_1$-$C_2$-alkyl or formyl;
$R_4$, $R_5$, $R_6$, $R_7$, $R_8$, $R_9$, $R_{10}$, $R_{11}$, $R_{12}$ and $R_{13}$, independently of one another, are hydrogen, fluorine or $CF_3$; and
$X_1$ and $X_2$ are O.

**22.** Ectoparasiticidal composition comprising a compound of the formula I as defined in any one of claims 1 to 20 and a physiologically acceptable carrier and/or dispersant.

**23.** Ectoparasiticidal composition according to claim 21 consisting of a pour-on or spot-on formulation.

**24.** Method of controlling ectoparasites, whereby an effective amount of at least one compound of formula I according to claim 1 according to claim 1 is administered to the habitat of the parasites.

**25.** Use of a compound of the formula I as defined in any one of claims 1 to 20 for the preparation of an ectoparasiticidal composition according to claim 21.

**26.** Compound of the formula I as defined in any one of claims 1 to 20 for the use in the treatment of ectoparasites on non-human animals.

European Patent Office

# PARTIAL EUROPEAN SEARCH REPORT

which under Rule 45 of the European Patent Convention shall be considered, for the purposes of subsequent proceedings, as the European search report

Application Number
EP 04 00 5423

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 0 650 482 B (SHELL INT RESEARCH) 3 May 1995 (1995-05-03)<br>* page 1, line 3 *<br>page 1, formula (I)<br>* page 5, line 12 - line 13 *<br>----- | 1-10 | C07D239/46<br>C07D239/60<br>C07D239/50 |
| X | WO 94/02470 A (SHELL INT RESEARCH ; MUNRO DAVID (GB); DAVIS ROYSTON (GB); DAY JANET A) 3 February 1994 (1994-02-03)<br>* page 1, line 1 - line 2 *<br>page 1, formula (I)<br>* page 7, line 28 *<br>* page 8, line 17 *<br>----- | 1-10 | |
| D,X | US 6 342 499 B1 (BLACK BRUCE CHRISTIAN ET AL) 29 January 2002 (2002-01-29)<br>* column 1, line 11 - line 17 *<br>* column 1, line 45 - line 55 *<br>column 2, formula (I)<br>----- | 1-10 | |

TECHNICAL FIELDS SEARCHED (Int.Cl.7)

C07D

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

2

| Place of search | Date of completion of the search | Examiner |
|---|---|---|
| Berlin | 1 November 2004 | Hoepfner, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

**INCOMPLETE SEARCH SHEET C**

Application Number
EP 04 00 5423

Although claims 1-10 are directed to a method of treatment of the human/animal body (Article 52(4) EPC), the search has been carried out and based on the alleged effects of the compound/composition.

-----

European Patent Office

Application Number
EP 04 00 5423

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☒ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☐ None of the further search fees have been paid within the fixed time limit. The present European search report has been drawn up for those parts of the European patent application which relate to the invention first mentioned in the claims, namely claims:

## ANNEX TO THE EUROPEAN SEARCH REPORT ON EUROPEAN PATENT APPLICATION NO. EP 04 00 5423

This annex lists the patent family members relating to the patent documents cited in the above-mentioned European search report.
The members are as contained in the European Patent Office EDP file on
The European Patent Office is in no way liable for these particulars which are merely given for the purpose of information.

01-11-2004

| Patent document cited in search report | Publication date | Patent family member(s) | Publication date |
|---|---|---|---|
| EP 0650482 B | 03-05-1995 | EP 0650482 A1 | 03-05-1995 |
| | | AT 206404 T | 15-10-2001 |
| | | AU 671845 B2 | 12-09-1996 |
| | | AU 4570093 A | 14-02-1994 |
| | | DE 69330882 D1 | 08-11-2001 |
| | | DE 69330882 T2 | 11-04-2002 |
| | | DK 650482 T3 | 03-12-2001 |
| | | JP 7508999 T | 05-10-1995 |
| | | JP 3353895 B2 | 03-12-2002 |
| | | MD 950235 A | 31-10-1997 |
| | | PL 307131 A1 | 02-05-1995 |
| | | PL 173066 B1 | 30-01-1998 |
| | | PL 173717 B1 | 30-04-1998 |
| | | PL 173091 B1 | 30-01-1998 |
| | | PL 173056 B1 | 30-01-1998 |
| | | PL 173467 B1 | 31-03-1998 |
| | | SK 3295 A3 | 10-05-1995 |
| | | US 5707995 A | 13-01-1998 |
| | | CA 2140346 A1 | 03-02-1994 |
| | | CN 1087085 A | 25-05-1994 |
| | | CZ 9500030 A3 | 13-09-1995 |
| | | EG 20267 A | 31-05-1998 |
| | | WO 9402470 A1 | 03-02-1994 |
| | | ES 2164666 T3 | 01-03-2002 |
| | | HU 70086 A2 | 28-09-1995 |
| | | IL 106324 A | 24-09-1998 |
| | | OA 10124 A | 18-12-1996 |
| | | PT 650482 T | 28-03-2002 |
| | | SG 49712 A1 | 15-06-1998 |
| | | TR 26781 A | 15-05-1995 |
| | | ZA 9305155 A | 20-06-1994 |
| WO 9402470 A | 03-02-1994 | AT 206404 T | 15-10-2001 |
| | | AU 671845 B2 | 12-09-1996 |
| | | AU 4570093 A | 14-02-1994 |
| | | CA 2140346 A1 | 03-02-1994 |
| | | CN 1087085 A | 25-05-1994 |
| | | CZ 9500030 A3 | 13-09-1995 |
| | | DE 69330882 D1 | 08-11-2001 |
| | | DE 69330882 T2 | 11-04-2002 |
| | | DK 650482 T3 | 03-12-2001 |
| | | EG 20267 A | 31-05-1998 |
| | | WO 9402470 A1 | 03-02-1994 |
| | | EP 0650482 A1 | 03-05-1995 |
| | | ES 2164666 T3 | 01-03-2002 |
| | | HU 70086 A2 | 28-09-1995 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 04 00 5423

This annex lists the patent family members relating to the patent documents cited in the above-mentioned European search report.
The members are as contained in the European Patent Office EDP file on
The European Patent Office is in no way liable for these particulars which are merely given for the purpose of information.

01-11-2004

| Patent document cited in search report | Publication date | Patent family member(s) | Publication date |
|---|---|---|---|
| WO 9402470 A | | IL 106324 A | 24-09-1998 |
| | | JP 7508999 T | 05-10-1995 |
| | | JP 3353895 B2 | 03-12-2002 |
| | | MD 950235 A | 31-10-1997 |
| | | OA 10124 A | 18-12-1996 |
| | | PL 307131 A1 | 02-05-1995 |
| | | PL 173066 B1 | 30-01-1998 |
| | | PL 173717 B1 | 30-04-1998 |
| | | PL 173091 B1 | 30-01-1998 |
| | | PL 173056 B1 | 30-01-1998 |
| | | PL 173467 B1 | 31-03-1998 |
| | | PT 650482 T | 28-03-2002 |
| | | SG 49712 A1 | 15-06-1998 |
| | | SK 3295 A3 | 10-05-1995 |
| | | TR 26781 A | 15-05-1995 |
| | | US 5707995 A | 13-01-1998 |
| | | ZA 9305155 A | 20-06-1994 |
| US 6342499 B1 | 29-01-2002 | NONE | |

EPO FORM P0459

For more details about this annex : see Official Journal of the European Patent Office, No. 12/82